(19) Europäisches Patentamt

European Patent Office

Office européen des brevets

(11) **EP 4 245 218 B1**

(12) **FASCICULE DE BREVET EUROPEEN**

(45) Date de publication et mention
de la délivrance du brevet:
**29.05.2024   Bulletin 2024/22**

(21) Numéro de dépôt: **22163111.2**

(22) Date de dépôt: **18.03.2022**

(51) Classification Internationale des Brevets (IPC):
**A61B 5/145** (2006.01)     **A61B 5/1455** (2006.01)
**A61B 5/00** (2006.01)     **G01N 21/27** (2006.01)
**G16H 50/20** (2018.01)

(52) Classification Coopérative des Brevets (CPC):
**A61B 5/14532; A61B 5/0095; A61B 5/1455;
A61B 5/7264; A61B 5/7267; G01N 21/274;
G16H 40/63;** A61B 5/0093; A61B 2560/0223

(54) **CAPTEUR NON INVASIF ET PROCÉDÉ DE MESURE**

NICHT-INVASIVER SENSOR UND MESSVERFAHREN

NON-INVASIVE SENSOR AND MEASURING METHOD

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(43) Date de publication de la demande:
**20.09.2023   Bulletin 2023/38**

(73) Titulaire: **Eclypia
38000 GRENOBLE (FR)**

(72) Inventeurs:
• **GALLEGOS, Alexandre
75002 Paris (FR)**
• **MONPEURT, Cyrielle
38000 Grenoble (FR)**
• **BLANC, Romain
38000 Grenoble (FR)**

(74) Mandataire: **Fidal Innovation
4-6 avenue d'Alsace
92400 Courbevoie (FR)**

(56) Documents cités:
EP-A1- 2 460 470          US-A1- 2003 023 152
US-A1- 2017 042 428

• **TRONSTAD CHRISTIAN ET AL: "Non-invasive
prediction of blood glucose trends during
hypoglycemia", ANALYTICA CHIMICA ACTA, vol.
1052, 15 décembre 2018 (2018-12-15), pages
37-48, XP085583751, ISSN: 0003-2670, DOI:
10.1016/J.ACA.2018.12.009**

**EP 4 245 218 B1**

**Description**

**DOMAINE DE L'INVENTION**

La présente invention se rapporte à un procédé de mesure et à un capteur non invasif permettant de mesurer un ou plusieurs paramètres d'intérêt dans un milieu cible.

Plus précisément, l'invention se rapporte à un capteur non invasif basé sur la détection d'un effet photothermique ou photoacoustique, notamment configuré pour mesurer des paramètres dans un milieu cible, tel qu'un milieu stratifié et/ou évolutif. Un paramètre mesuré peut par exemple être la glycémie dans la peau.

**ARRIÈRE-PLAN TECHNOLOGIQUE**

Dans le domaine des capteurs pour les organismes vivants, il est connu de réaliser des capteurs non invasifs basés sur la détection photoacoustique ou photothermique.

Une zone d'intérêt d'un milieu à analyser, appelée cible, est irradiée au moyen d'un faisceau laser de longueur d'onde et de fréquence de modulation choisies en fonction du paramètre d'intérêt à mesurer. Le faisceau laser est absorbé par la cible sur une profondeur qui dépend de la structuration de la cible. L'absorption d'énergie lumineuse entraîne un échauffement local de la cible. En réaction à cet échauffement, une onde thermique de fréquence égale à la fréquence de modulation du laser est générée dans la cible. Cette onde se propage dans la cible et notamment jusqu'à la surface extérieure de la cible.

L'onde thermique peut être directement détectée et analysée. On parle alors de photothermie. La détection photoacoustique exploite quant à elle le fait que l'onde thermique est associée à une onde de pression de fréquence identique à la fréquence de modulation.

Dans le cas de la détection photoacoustique indirecte, on détecte l'onde de pression générée dans le milieu extérieur fluide lorsque l'onde thermique générée dans la cible parvient, après propagation, à l'interface cible - milieu extérieur fluide.

Cet effet photoacoustique a fait l'objet de nombreuses études théoriques. Allan Rosencwaig et Allen Gersho ont notamment développé un modèle théorique du signal photoacoustique. Ce modèle met en jeu les propriétés physico-chimiques de l'échantillon à analyser, parmi lesquelles la longueur de diffusion optique, l'épaisseur et la longueur de diffusion thermique de l'échantillon. (Rosencwaig, A. and Gersho, A. (1976), Theory of the photoacoustic effect with solids, Journal of Applied Physics, 47, 64).

Hu et al. ont quant à eux développé une théorie généralisée de l'effet photoacoustique dans un matériau stratifié. (Hu, H., Wang, X., & Xu, X. (1999). Generalized theory of the photoacoustic effect in a multilayer material. Journal of Applied Physics, 86, 3953-3958.)

La détection photoacoustique présente de nombreux avantages par rapport à d'autres techniques de détection parmi lesquels on peut citer l'aspect orthogonal de la transduction : le signal optique en entrée du milieu à analyser est converti en un signal acoustique qui est très spécifique du phénomène à observer et qui permet d'utiliser des capteurs peu coûteux et miniaturisés.

La difficulté de la détection photoacoustique ou photothermique provient entre autres:

- du nombre de paramètres influençant en général le signal détecté et,
- pour certains analytes d'intérêt présents en concentration faible dans le milieu à analyser, de la faible proportion du signal détecté spécifique à chacun de ces paramètres d'intérêt.

Dans un matériau stratifié, pour pouvoir déduire du signal photoacoustique ou photothermique la concentration d'une couche donnée en un analyte d'intérêt, il est ainsi nécessaire de connaître tous les autres paramètres influençant ce signal. En particulier, il est nécessaire de connaître la structuration du matériau, c'est-à-dire les épaisseurs des différentes couches constituant le matériau, leurs compositions physico-chimiques (à l'exception du paramètre d'intérêt à mesure), ainsi qu'éventuellement leurs conductivités thermiques ou encore la résistance thermique associée à chaque interface entre deux couches successives.

L'étalonnage une fois pour toutes (ou tout au moins pour une durée d'utilisation significative) d'un capteur basé sur la détection photoacoustique ou photothermique n'est possible que si seul le paramètre d'intérêt varie dans la cible. En revanche, lorsque les caractéristiques du milieu stratifié à analyser varient, il est nécessaire de procéder à un étalonnage du capteur régulièrement pour obtenir une mesure dont la précision est acceptable. Ce problème se pose plus particulièrement pour les capteurs destinés à être utilisés sur des organismes vivants. Par exemple, dans le cas d'un capteur non invasif de glycémie interstitielle, l'étalonnage d'un capteur non invasif basé sur la détection photoacoustique ou photothermique ne peut être obtenu qu'avec une précision limitée car la composition de la peau varie non seulement d'un patient à l'autre mais aussi au cours du temps pour un patient donné.

Le document EP2460470 décrit un procédé de calibration régulière pour un capteur de glycémie non invasif comprenant une source laser dans l'infrarouge proche. On note que ce capteur utilise une méthode de détection autre que la détection photoacoustique ou photothermique, puisque c'est la fraction de l'onde lumineuse incidente qui est transmise ou diffusée par le matériau qui est détectée. Les contraintes techniques et limitations d'un tel capteur, notamment en termes de consommation énergétique et de précision de la mesure, ne sont donc pas les mêmes que celles d'un capteur basé sur la détection photoacoustique ou photothermique.

Dans le procédé de calibration de EP2460470, une pluralité de modèles de calibration ou une pluralité de jeux de données pour créer ces modèles, éventuellement obtenus par simulation numérique, sont stockés en mémoire du capteur.

Au moment de la calibration, un spectre optique du tissu à analyser, appelé spectre de référence, est mesuré par irradiation du tissu sur une gamme de longueurs d'onde prédéterminée, sur la base duquel un modèle de calibration est choisi pour les mesures à suivre.

A une date d'analyse ultérieure, le spectre optique du tissu à analyser est à nouveau mesuré et une différence entre le spectre mesuré et le spectre de référence est calculée. La qualité du modèle de calibration précédemment choisi est évaluée sur la base de cette différence et si cette évaluation est défavorable, un nouveau modèle de calibration est choisi pour déterminer la valeur de la glycémie mesurée. La valeur de la glycémie est alors déterminée par le capteur en substituant les absorbances mesurées à chaque longueur d'onde du spectre dans le modèle de calibration.

L'adaptation du modèle de calibration au fur et à mesure du temps suivant le procédé de EP2460470 permet de mesurer la glycémie avec une précision accrue par rapport aux capteurs de glycémie dont la calibration est faite une seule fois lors de l'adaptation du capteur au patient. L'inconvénient du procédé de EP2460470 est qu'il nécessite pour chaque recalibration l'acquisition d'un spectre optique du tissu à analyser sur une gamme complète de longueurs d'onde. Cette acquisition est associée à une consommation énergétique importante.

En conséquence, si l'on adapte purement et simplement le procédé de EP2460470 à un capteur continu basé sur la détection photoacoustique indirecte, le coût en énergie de ce procédé n'est pas tenable ou nécessite une batterie dont le poids est difficilement acceptable pour un patient qui serait équipé d'un tel capteur.

En outre, la détection photoacoustique nécessite non seulement un choix de longueur d'onde mais aussi un choix de la fréquence de modulation du laser. En effet, la profondeur de pénétration du laser dans la cible dépend de la fréquence de modulation du laser. Si la structure de la cible (par exemple la peau) change au fur et à mesure du temps, la fréquence de modulation à utiliser pour mesurer le paramètre d'intérêt (par exemple la glycémie interstitielle) change elle aussi au cours du temps. Pour mettre en oeuvre le procédé de calibration de EP2460470 sur un capteur basé sur la détection photoacoustique ou photothermique, il serait donc nécessaire non seulement de faire varier la longueur d'onde du laser comme précédemment pour pouvoir calculer la différence entre le spectre de référence et le spectre calibré mais aussi la fréquence de modulation du laser. En d'autres termes, il faudrait obtenir avant chaque mesure (ou chaque série de mesure) des séries de spectres optiques de la cible à des fréquences de modulation variées. Le problème de consommation énergétique serait donc encore accru si l'on réalisait une simple transposition du procédé de EP2460470 à un capteur non invasif basé sur la détection photoacoustique indirecte.

Enfin, EP2460470 pose un modèle de peau a priori, composé de trois couches : une couche de surface de 0,1 mm d'épaisseur, une couche interne de 0,9 mm d'épaisseur et une couche subcutanée de 2,0 mm d'épaisseur. Même si EP2460470 évoque la possibilité (au §79) d'utiliser l'épaisseur (globale) de la peau comme une variable dans les simulations, il ne fait pas la preuve de la faisabilité d'un tel mode de réalisation ni n'indique s'il est possible de tenir compte d'un nombre de couches différent de trois. Le procédé de calibrage de EP2460470 ne semble donc pas apte à tenir compte de toute la variabilité intrinsèque du milieu stratifié à étudier. L'invention vise donc à améliorer la précision d'un capteur non invasif dans un milieu cible, notamment stratifié et/ou évolutif basé sur la détection photoacoustique ou photothermique tout en contrôlant la consommation énergétique de ce capteur ou encore à réduire la consommation énergétique de ce capteur tout en contrôlant sa précision.

**RÉSUMÉ DE L'INVENTION**

Ainsi, l'invention se rapporte à un procédé de mesure d'un paramètre d'intérêt dans un milieu cible au moyen d'un capteur non invasif basé sur la détection photoacoustique ou la détection photothermique, comprenant :

a) on fournit un capteur comprenant :

- une source de lumière,
- un dispositif de contrôle des paramètres d'irradiation de la source de lumière,
- une cellule de détection configurée pour détecter un signal acoustique ou thermique,
- une mémoire dans laquelle est stockée une table de correspondance comprenant des configurations modèles chacune représentative d'un état donné du milieu cible et des cas d'irradiation optimaux comprenant chacun un jeu de paramètres d'irradiation, chaque configuration modèle étant associée à un cas d'irradiation optimal,
- et un module d'adaptation échangeant des informations avec la cellule de détection et le dispositif de contrôle des paramètres d'irradiation de la source de lumière, le module d'adaptation comprenant un processeur adapté pour implémenter un algorithme de modélisation inverse recevant en entrée un cas d'irradiation comprenant un jeu de paramètres d'irradiation et un signal acoustique ou thermique et fournissant en sortie une configuration modèle et une valeur du paramètre d'intérêt ;

b) le module d'adaptation choisit une configuration modèle d'irradiation initiale ;

c) le module d'adaptation détermine dans la table de correspondance le cas d'irradiation optimal pour la configuration modèle d'irradiation choisie;

d) la source de lumière irradie le milieu cible suivant le jeu de paramètres d'irradiation dudit cas d'irradiation optimal ;

e) la cellule de détection détecte un signal acoustique ou thermique généré en réponse à l'irradiation ;

f) le processeur du module d'adaptation implémente l'algorithme de modélisation inverse, reçoit en entrée le signal acoustique ou thermique détecté par la cellule de détection et le cas d'irradiation optimal utilisé pour l'irradiation, et renvoie en sortie une configuration modèle en cours et une valeur du paramètre d'intérêt estimée ;

g) le processeur du module d'adaptation évalue la configuration modèle d'irradiation choisie par comparaison avec la configuration modèle en cours, et seulement si cette évaluation est défavorable : g1)le module d'adaptation (14) reçoit en entrée la configuration modèle en cours (CMmes) et renvoie en sortie une nouvelle configuration modèle en vue d'une irradiation (CMirrad), puis on réitère c), d), e) et f) ;

h) la valeur du paramètre d'intérêt mesurée (Pmes) par le capteur est la dernière valeur du paramètre d'intérêt estimée (Pest).

Grâce à ces dispositions, il est possible d'obtenir une mesure de glycémie avec :

- soit une seule irradiation (pas d'exécution de la sous-étape g1, dans le cas d'une évaluation favorable). Cette irradiation est alors effectuée selon un cas d'irradiation choisi par défaut mais correspondant à une consommation énergétique contrôlée (les paramètres d'irradiation étant par exemple choisis en nombre limité). L'étape g) d'évaluation de la configuration modèle d'irradiation permet d'acquérir la certitude que la précision de la mesure était bien la plus élevée possible pour le cas d'espèce au vu des cas d'irradiations disponibles dans la table de correspondance, ce qui est donc un avantage par rapport à un procédé dans lequel l'étape g) n'est pas prévue et pour lequel on ne dispose pas de cette certitude ;

- soit deux irradiations (étape g avec exécution de la sous-étape g1, dans le cas d'une évaluation défavorable) et donc comprenant une réitération de c), d), e) et f)). La seconde irradiation est alors effectuée selon le cas d'irradiation permettant d'obtenir la précision la plus élevée possible pour le cas d'espèce au vu des informations supplémentaires acquises grâce à la première irradiation et des cas d'irradiation disponibles dans la table de correspondance. La précision du procédé est donc connue et améliorée par rapport à un procédé ne présentant pas l'étape g).

Le procédé permet donc dans le premier cas une validation des paramètres d'irradiation choisi et d'acquérir la certitude que la précision de la mesure est la meilleure possible pour cette configuration de capteur et l'état de la cible à la date de la mesure compte tenu des informations supplémentaires acquises lors de l'irradiation, et dans le second cas, une adaptation des paramètres d'irradiation pour obtenir dans un second temps la meilleure précision de mesure possible compte tenu des informations supplémentaires acquises lors de la première irradiation, le tout étant effectué avec une consommation énergétique contrôlée et notamment sans nécessiter l'acquisition de spectres complet ni en fréquence d'irradiation ni en fréquence de modulation. On note que la table de correspondance est essentielle pour ce procédé. C'est cette table de correspondance qui permet d'associer à chaque configuration modèle en cours ou choisie pour l'irradiation le cas d'irradiation le plus adapté et donc de limiter la consommation énergétique pour chaque irradiation.

Selon différents aspects, il est possible de prévoir l'une et/ou l'autre des caractéristiques ci-dessous prises seules ou en combinaison.

Selon une réalisation, g1) comprend on réitère g) à l'issue de f). Dans ce cas, l'irradiation est répétée tant que les paramètres d'irradiation utilisés ne sont pas les paramètres d'irradiation optimaux pour l'état de la cible à la date de la mesure.

Ce mode de réalisation permet, notamment si la répétition de l'irradiation est faite à une fréquence supérieure à la fréquence caractéristique des modifications de la structure de la cible, de converger de proche en proche vers les paramètres d'irradiation permettant d'obtenir la meilleure précision. Encore une fois, la consommation énergétique pour chaque irradiation étant contrôlée du fait que le nombre de paramètres d'irradiation est limité pour chaque cas d'irradiation, même si la convergence n'est obtenue qu'après trois, quatre, cinq voire dix irradiations successives, la consommation énergétique globale peut être maîtrisée et notamment inférieure à celle qui serait nécessaire pour l'acquisition d'un spectre complet en fréquence d'irradiation at/ou en fréquence de modulation, et ce tout en contrôlant, voire en améliorant simultanément la précision de la mesure.

Selon une réalisation du processus, on génère au préalable une table de correspondance au moyen d'un processeur et d'une base de données de configurations modèles comprenant des multiplets (configuration modèle, cas d'irradiation , paramètre d'intérêt) et un signal acoustique ou thermique détecté par la cellule de détection associé à chaque multiplet et on stocke cette table de correspondance dans la mémoire du capteur non invasif.

Grâce à cette disposition, la table de correspondance est obtenue de manière automatisée et peut par exemple être actualisée lorsque la base de données de configurations modèles s'enrichit ou doit être adaptée à un patient ou à un type de patients donnés.

Selon un mode de réalisation, le procédé de mesure comprend :
un processeur apprend au moins un algorithme de modélisation inverse à partir de la base de données de configurations modèles et on stocke l'au moins un algorithme de modélisation inverse dans la mémoire du capteur non invasif.

Grâce à cette disposition, un ou plusieurs algorithmes de modélisation inverse peuvent être appris de manière automatisée, par exemple chacun adapté à une configuration modèle et un cas d'irradiation donné ou à un groupe de configurations modèles et de cas d'irradiation donnés. Selon un mode de réalisation du procédé, au moins une partie des signaux acoustiques ou thermiques détectés par la cellule de détection associés aux multiplets stockés dans la base de données de configurations modèles sont simulés, c'est-à-dire qu'ils sont générés au moyen d'un dispositif informatisé de simulation.

Grâce à cette disposition, il est possible de générer un grand nombre de multiplets, par exemple correspondant à des états du milieu cible aussi variés que possible et à des cas d'irradiation, eux aussi, aussi variés que possible, de manière beaucoup plus rapide et moins coûteuse que si ces multiplets étaient obtenus par l'expérience en situation réelle. Dans le cas où le milieu cible est un tissu d'un être vivant, tel que la peau, il est possible de générer des multiplets correspondant à des situations physiologiques rares, extrêmes ou simplement plus variées que celles qui seraient accessibles avec un ensemble de patients tests. Une table de correspondance générée à partir d'une telle base de données peut donc couvrir une gamme de situations beaucoup plus étendues avec un grain beaucoup plus fin qu'en l'absence de simulation, ce qui permet au final de générer des cas d'irradiation optimaux d'autant mieux adaptés en termes de consommation et/ou de précision à chaque configuration modèle.

L'invention porte en outre sur un capteur non invasif basé sur la détection photoacoustique ou photothermique configuré pour mesurer un paramètre d'intérêt dans un milieu cible comprenant :

- une source de lumière,
- un dispositif de contrôle des paramètres d'irradiation de la source de lumière,
- une cellule de détection configurée pour détecter un signal acoustique ou thermique,
- une mémoire dans laquelle est stockée une table de correspondance comprenant des configurations modèles chacune représentative d'un état donné du milieu cible et des cas d'irradiation optimaux comprenant chacun un jeu de paramètres d'irradiation, chaque configuration modèle étant associée à un cas d'irradiation optimal,

le capteur non invasif comprenant de plus un module d'adaptation adapté pour échanger des informations avec la cellule de détection et le dispositif de contrôle des paramètres d'irradiation de la source de lumière, le module d'adaptation comprenant un processeur adapté pour implémenter un algorithme de modélisation inverse recevant en entrée un cas d'irradiation comprenant un jeu de paramètres d'irradiation et un signal acoustique ou thermique et fournissant en sortie une configuration modèle et une valeur du paramètre d'intérêt,
le module d'adaptation étant en outre configuré pour :

- choisir une configuration modèle d'irradiation (CMirrad) initiale,
- déterminer dans la table de correspondance un cas d'irradiation optimal correspondant à une configuration modèle d'irradiation,
- transmettre un cas d'irradiation optimal au dispositif de contrôle des paramètres d'irradiation de la source de lumière,
- recevoir un signal détecté par la cellule de détection,
- déterminer une configuration modèle en cours et une valeur du paramètre d'intérêt estimée sur la base d'un signal photoacoustique ou photothermique détecté reçu et d'un cas d'irradiation optimal,
- évaluer une configuration modèle d'irradiation par comparaison avec une configuration modèle en cours,
- déterminer une nouvelle configuration modèle d'irradiation lorsqu'il reçoit une configuration modèle en cours,
- déterminer la valeur du paramètre d'intérêt mesurée sur la base de la dernière valeur du paramètre d'intérêt estimée.

Grâce à cette disposition, le capteur peut mesurer avec une précision et/ou une consommation contrôlées un paramètre d'intérêt dans un milieu cible, notamment un tissu d'un être vivant. L'invention concerne enfin un programme d'ordinateur comprenant des instructions qui conduisent le capteur non invasif suivant le mode de réalisation précédent à exécuter les étapes du procédé suivant l'un quelconque des modes de réalisation décrit plus haut.

**BRÈVE DESCRIPTION DES DESSINS**

Des modes de réalisation de l'invention seront décrits ci-dessous par référence aux dessins, décrits brièvement ci-dessous :

La figure 1 représente une modélisation type d'un milieu cible stratifié de type peau.
La figure 2 représente les étapes d'un procédé de mesure optimisé dans un mode de réalisation particulier de l'invention, dans le cas où l'on souhaite mesurer la glycémie interstitielle d'un patient.

La figure 3 représente les éléments principaux d'un capteur 1 selon l'invention, le capteur 1 étant dans ce cas positionné en contact avec le milieu cible 2. La figure 4 représente les étapes mise en oeuvre par le module de simulation 15 pour simuler un signal photoacoustique qui serait détecté par une cellule photoacoustique donnée en réponse à l'irradiation d'un milieu stratifié cible modélisé au moyen des paramètres de la configuration modèle CMk suivant les paramètres d'irradiation du cas d'irradiation Ij. La figure 5 représente le fonctionnement d'un modèle inverse.

Les figures 6a, 6b et 6c représentent les résultats d'une analyse d'influence des variables de type SHAP (« SHapley Additive exPlanations ») effectuées sur trois modèles inverses entraînés respectivement sur :

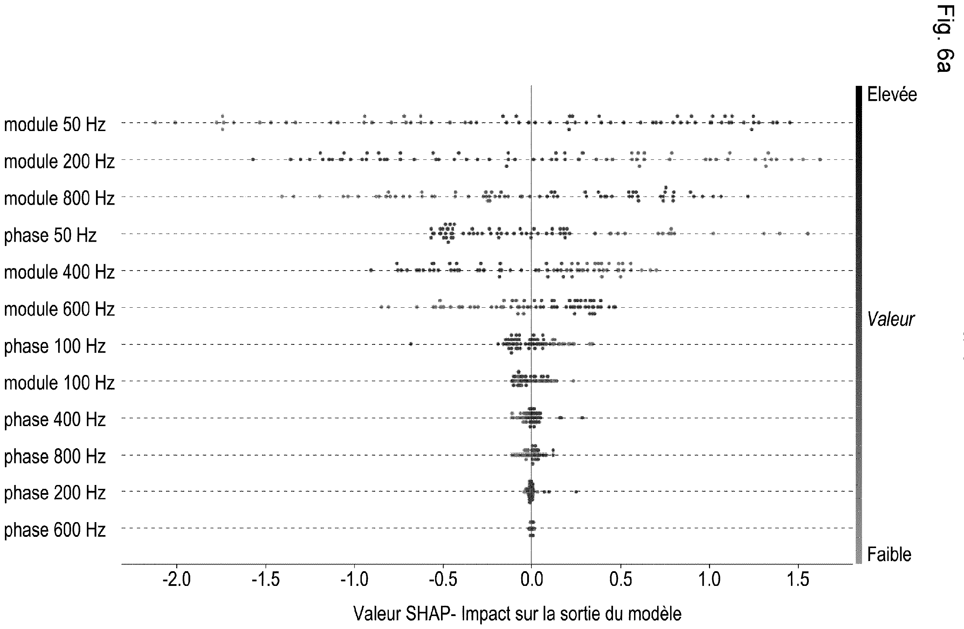

- figure 6a : un premier groupe « stratum épais », qui correspond à des configurations modèles de peau bicouches dans lesquelles l'épaisseur de la couche supérieure, modélisant le stratum corneum, est supérieure à 18 μm ;

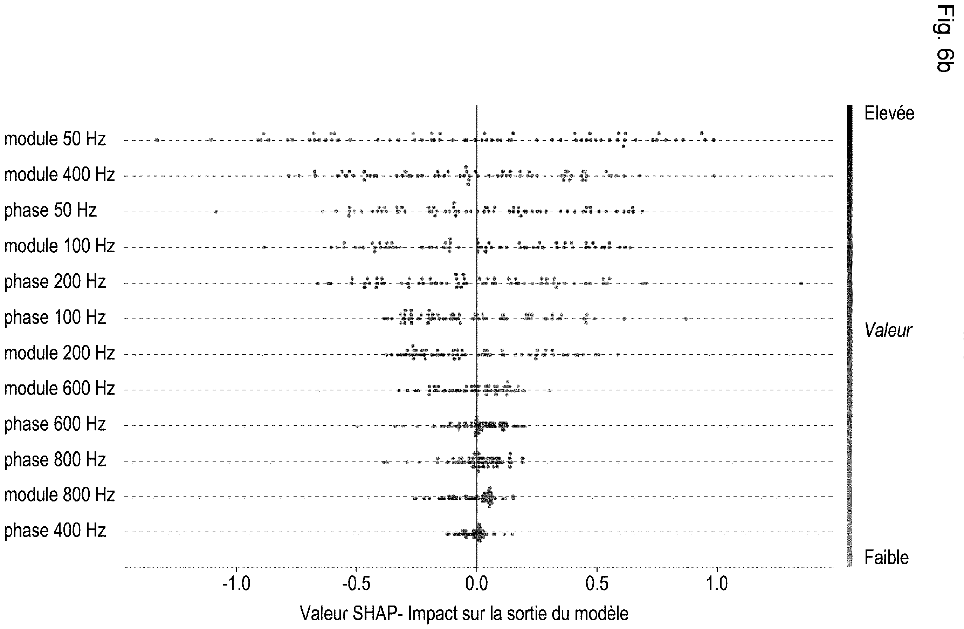

- figure 6b : un second groupe « stratum fin » qui correspond à des configurations modèles de peau bicouches dans lesquelles l'épaisseur de la couche supérieure, modélisant le stratum corneum, est inférieure à 18 μm ;

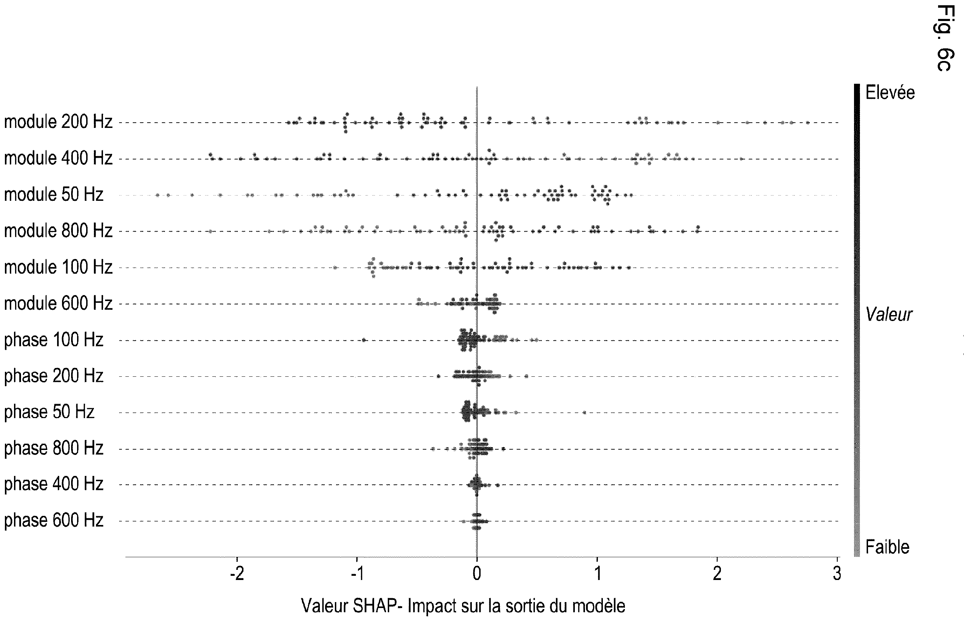

- figure 6c l'ensemble du premier et du second groupe ;

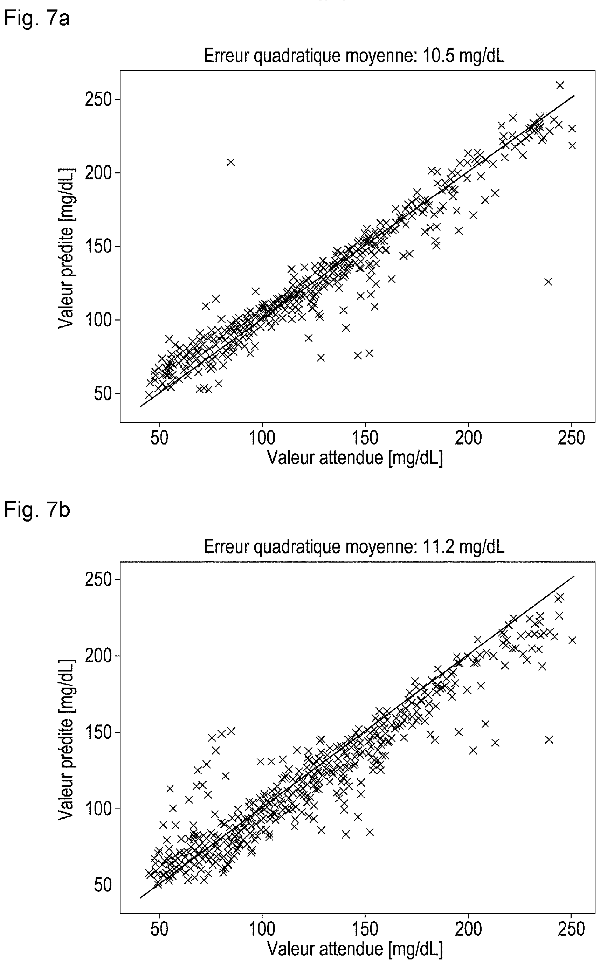

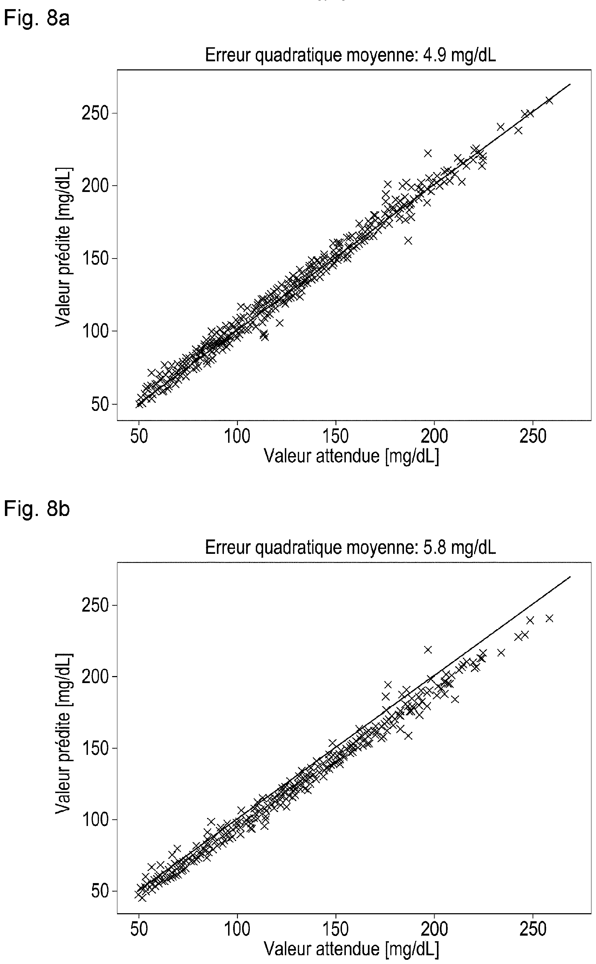

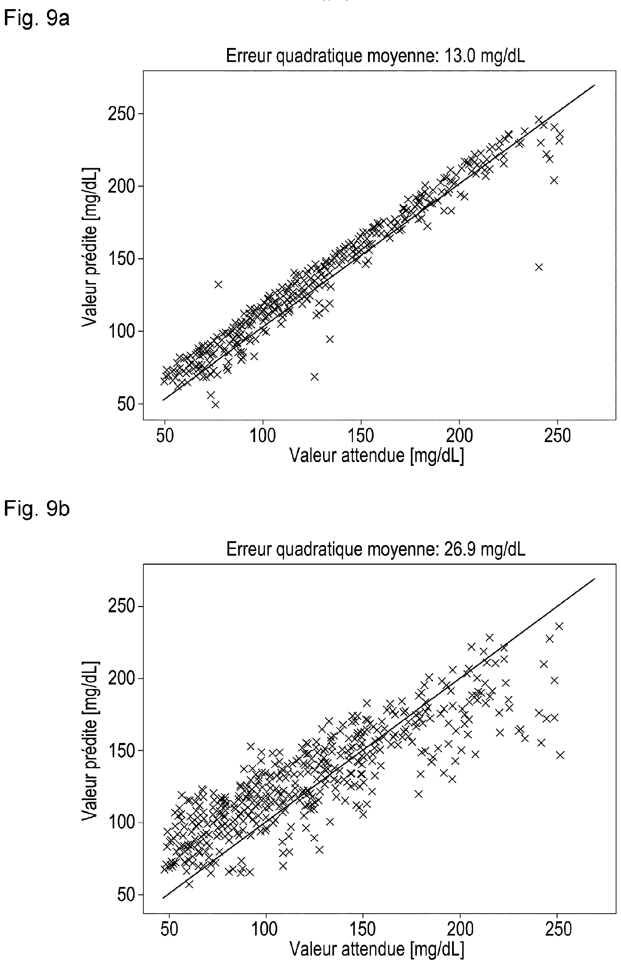

chacun de ces modèles étant entraîné à partir des signaux détectés suite à une irradiation suivant six fréquences de modulation différentes. Sur les figures 6a, 6b et 6c, l'axe des abscisses représente la valeur SHAP. Chaque point d'une figure correspond à une valeur de Shapley pour une variable et une instance. La position sur l'axe vertical est déterminée par la variable et sur l'axe des abscisses par la valeur de Shapley. L'intensité (en nuances de gris) couleur représente la valeur de la variable. La figure 7a représente la valeur de glycémie prédite par un premier modèle inverse, entraîné sur le premier groupe « stratum épais » à partir des signaux détectés suite à une irradiation suivant six fréquences de modulation différentes. La figure 7b représente la valeur de glycémie prédite par un deuxième modèle inverse, entraîné sur le premier groupe « stratum épais » à partir des signaux détectés suite à une irradiation suivant les deux variables les plus influentes pour ce groupe, identifiées sur la figure 6a. La figure 8a représente la valeur de glycémie prédite par un troisième modèle inverse, entraîné sur le second groupe « stratum fin » à partir des signaux détectés suite à une irradiation suivant six fréquences de modulation différentes. La figure 8b représente la valeur de glycémie prédite par un quatrième modèle inverse, entraîné sur le second groupe « stratum fin » à partir des signaux détectés suite à une irradiation suivant les deux variables les plus influentes pour ce groupe « stratum fin », identifiées sur la figure 6b. La figure 9a représente la valeur de glycémie prédite par un cinquième modèle inverse, entraîné sur l'ensemble des deux groupes « stratum fin » et « stratum épais » à partir des signaux détectés suite à une irradiation suivant six fréquences de modulation différentes. La figure 9b représente la valeur de glycémie prédite par un sixième modèle inverse, entraîné sur l'ensemble des deux groupes « stratum fin » et « stratum épais » à partir des signaux détectés suite à une irradiation suivant les deux variables les plus influentes pour cet ensemble des deux groupes « stratum fin » et « stratum épais » identifiées sur la figure 6c.

Sur les dessins, des références identiques désignent des objets identiques ou similaires.

## DESCRIPTION DÉTAILLÉE

L'invention concerne un capteur non invasif 1 d'un ou plusieurs paramètres d'un milieu cible 2, notamment d'un milieu cible stratifié 2 dont la structuration peut éventuellement évoluer au cours du temps. Le milieu cible stratifié 2 peut être, à titre d'exemple, un tissu d'un organisme humain ou animal, tel que la peau.

Les paramètres à mesurés sont appelés dans la suite « paramètres d'intérêt ».

Un paramètre d'intérêt peut notamment être un paramètre physiologique dans le cas où le milieu stratifié est un tissu d'un être humain ou d'un animal.

Par exemple, le paramètre physiologique à mesurer est la glycémie, notamment la glycémie interstitielle. Il pourrait aussi s'agir de mesurer la teneur en eau dans une couche particulière de la peau ou encore la concentration en lactate d'une couche particulière. Ces exemples ne sont pas limitatifs.

Le capteur 1 peut être portable et il peut permettre le suivi en continu du ou des paramètres d'intérêt.

Le capteur non invasif 1 peut être basé sur la détection photoacoustique ou la détection photothermique.

Le procédé de mesure est particulièrement adapté pour améliorer la précision d'un capteur non invasif basé sur la détection photoacoustique indirecte, pour laquelle le capteur détecte une onde acoustique générée dans le gaz environnant le milieu cible 2 en réponse à une irradiation, tout en contrôlant sa consommation énergétique ou encore pour réduire la consommation énergétique du capteur tout en contrôlant sa précision. Toutefois, il est tout-à-fait possible de mettre en oeuvre ce procédé sur un capteur non invasif basé sur la photothermie ou encore sur la détection photoacoustique directe pour obtenir l'un des deux effets techniques précédents. Pour simplifier la compréhension, l'exemple de la détection photoacoustique indirecte sera décrit plus en détails ci-après, mais la généralisation à un capteur basé sur la photoacoustique indirecte ou sur la photothermie sera faite sans difficulté.

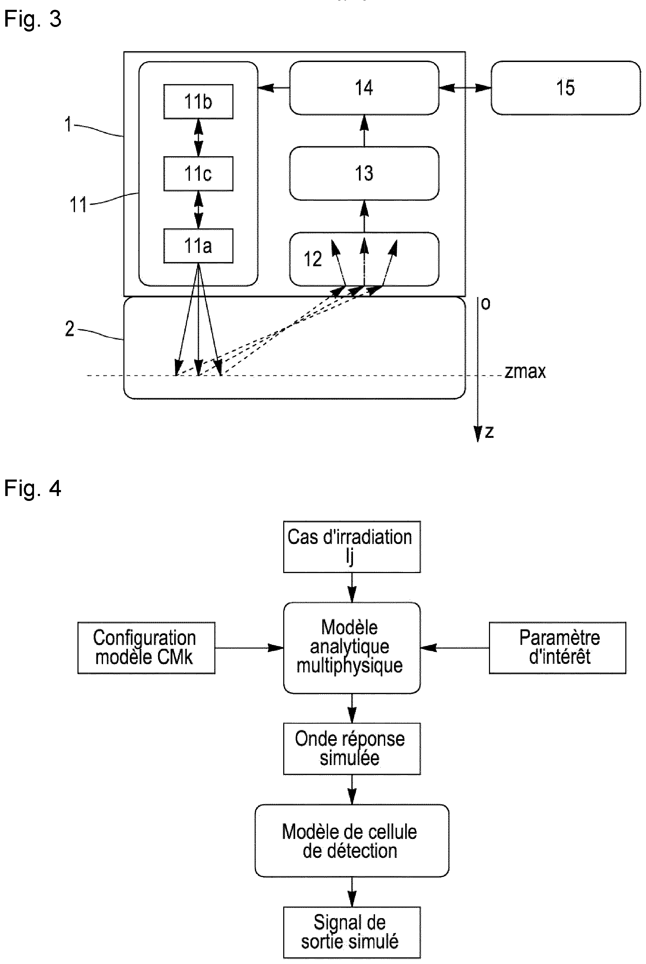

Le capteur non invasif 1 est représenté schématiquement sur la figure 3. Il comprend :

- un dispositif d'irradiation 11 comprenant une source de lumière 11a, un dispositif de modulation de L'intensité de cette source de lumière 11b, un dispositif de contrôle 11c d'au moins une fréquence de modulation à laquelle le dispositif de modulation d'intensité 11b module l'intensité d'une lumière émise par la source de lumière ;
- au moins une cellule de détection photoacoustique 12 détectant un signal généré en réponse à une irradiation d'un milieu cible 2 par la lumière émise, par exemple détectant directement ou indirectement une onde thermique qui se propage dans un milieu cible 2 ;
- un module de traitement du signal 13 configuré pour recevoir et traiter des données de l'au moins une cellule de détection 12 ;
- un module d'adaptation 14 des paramètres d'irradiation et du modèle de calibration ;
- un module de simulation 15, distant ou embarqué avec les autres éléments du capteur non invasif 1.

Dans un mode de réalisation particulier, la source de lumière 11a émet un faisceau laser modulé en intensité à au moins une longueur d'onde particulière vers le milieu cible 2.

La source de lumière 11a peut notamment être une diode électroluminescente (DEL), ou une puce laser. La source de lumière 11a peut, en outre ou en variante, comprendre un laser à cascade quantique (QCL) émettant dans la région de l'infrarouge moyen (MIR-QCL), un laser ICL (« interband cavity laser », un laser à cavité interne ou externe, un laser GaSb. Ces exemples ne sont pas limitatifs. La source de lumière 11a pourra être choisie en fonction du milieu cible 2 et/ou des paramètres d'intérêt.

Le capteur non invasif 1 peut comprendre plusieurs sources de lumière 11a.

Le capteur non invasif 1 comprend également les circuits associés à la (ou aux) sources de lumière 11a et au moins un dispositif de contrôle 11c configuré pour contrôler la fréquence à laquelle au moins un dispositif de modulation d'intensité 11b module l'intensité d'au moins une source de lumière 11a, de sorte que l'intensité de la lumière émise par la source de lumière 11a soit modulée à une fréquence de modulation réglable. La fréquence de modulation de l'intensité d'une source de lumière 11a donnée est appelée fmod dans la suite de la description.

La source de lumière 11a peut être modulée en intensité par tout moyen électrique ou mécanique connu.

La lumière peut être émise par une source de lumière 11a donnée de manière continue ou pulsée.

La lumière incidente sur le milieu cible 2, émise par la (ou les) source(s) de lumière 11a modulée(s) en intensité, se propage jusqu'au milieu cible 2 puis à travers ce milieu cible 2 (phénomène symbolisé par des flèches en traits pleins sur la figure 3). Elle est alors progressivement absorbée par les différents constituants de cette cible 2, sur une profondeur zmax qui dépend de la structure du milieu cible 2 et de sa composition physico-chimique. L'absorption de l'énergie lumineuse provoque un échauffement local du milieu cible 2. En conséquence, une onde thermique de fréquence égale à la fréquence de modulation de la source de lumière se propage dans le milieu cible 2 (phénomène symbolisé par des flèches en traits pointillés sur la figure 3), notamment vers la surface du milieu cible 2. Cette onde thermique donne naissance dans le milieu gazeux extérieur à la cible à une onde de pression de même fréquence, qui se propage dans ce milieu gazeux entourant le milieu cible 2 et notamment dans la cellule de détection photoacoustique 12 (phénomène symbolisé par des flèches en pointillés alternés sur la figure 3).

La cellule de détection 12 comprend dans le cas de la détection photoacoustique une chambre remplie d'un gaz (par exemple de l'air) à travers lequel se propage l'onde acoustique, et un ou plusieurs capteurs appropriés placés dans cette chambre, par exemple face au milieu cible 2. Il s'agit par exemple d'un ou plusieurs capteurs électroacoustiques configurés pour convertir la pression de l'onde acoustique en un signal électrique, tel qu'un microphone ou encore un transducteur piézoélectrique.

Chaque capteur électroacoustique est fonctionnellement connecté au module de traitement de signal 13.

Le module de traitement de signal 13 peut comprendre un convertisseur analogique-numérique configuré pour convertir le signal électrique analogique du capteur électroacoustique en un signal numérique.

Le module de traitement du signal 13 peut comprendre un dispositif de détection synchrone adapté pour démoduler et extraire le signal d'intérêt du signal détecté.

Le module de traitement de signal 13 comprend éventuellement un amplificateur opérationnel connecté de manière opérationnelle au convertisseur analogique-numérique et configuré pour amplifier le signal électronique dérivé de la réponse acoustique de la cible 2 transmis par un capteur électroacoustique.

Dans des exemples de modes de réalisation, le convertisseur analogique-numérique est fonctionnellement connecté à un processeur de signal numérique pour le traitement du signal numérique.

Le capteur non invasif 1 selon l'invention comprend en outre un module d'adaptation 14 des paramètres d'irradiation et du modèle de calibration, ainsi qu'un module de simulation 15. Ces deux éléments sont décrits dans les parties qui suivent.

**Module d'adaptation 14**

Le module d'adaptation 14 est un dispositif informatisé comprenant au moins un processeur

- qui peut échanger des informations avec le module de simulation 15 décrit plus loin ;
- qui peut recevoir des informations de la cellule de détection 12 et/ou du module de traitement du signal 13 le cas échéant;
- et qui peut transmettre des informations au(x) dispositif(s) de contrôle 11c d'au moins un paramètre d'irradiation de la source de lumière 11a (par exemple une fréquence de modulation à laquelle le dispositif de modulation d'intensité 11b module l'intensité d'une lumière émise par la source de lumière 11a, le nombre d'onde d'une lumière émise par la source de lumière 11a, la puissance lumineuse de la source de lumière 11a, ...).

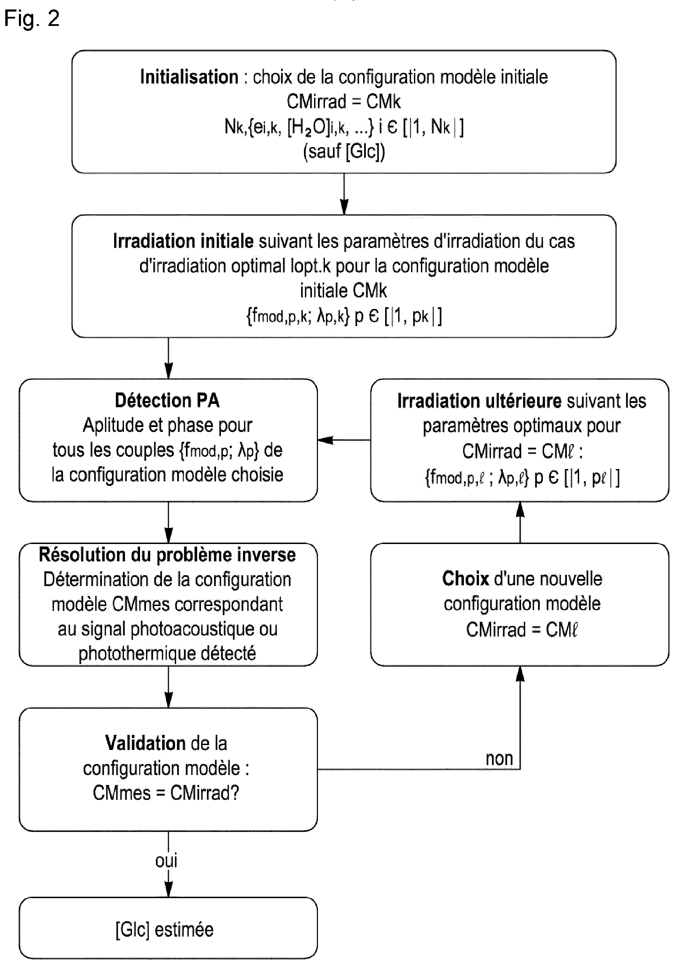

Les étapes mises en oeuvre par un capteur non invasif 1 comprenant un module d'adaptation 14 pour une mesure du paramètre d'intérêt sont représentées schématiquement sur la figure 2 dans le cas particulier où le milieu cible 2 est la peau (il s'agit donc dans ce cas d'un milieu stratifié) et le paramètre d'intérêt est la glycémie interstitielle.

Le capteur non invasif 1 comprend en outre une mémoire de stockage d'une base de données de configurations modèles, d'une table de correspondance et d'un ou plusieurs modèles inverses qui sont décrits ci-après.

Cette mémoire de stockage peut être distribuée et/ou partagée dans/avec le module d'adaptation (14) et/ou le module de simulation (15).

Le module d'adaptation 14 réalise les étapes du procédé qui permettent de choisir la configuration modèle de milieu cible 2 la plus adaptée au milieu cible 2 à la date de la mesure dans la base de données de configurations modèles, de choisir les paramètres d'irradiation optimaux pour la mesure sur la base de la table de correspondance et de déterminer le modèle inverse le plus adapté, c'est-à-dire le plus précis, pour le calcul du paramètre mesuré à partir du signal détecté pour cette mesure particulière. Nous décrirons ces étapes après avoir décrit les étapes permettant de générer la base de données de configurations modèles et la table de correspondance.

La base de données de configurations modèles et la table de correspondance sont générées à partir d'un module de simulation 15, embarqué dans le capteur 1 ou distant. Dans le cas où le module de simulation 15 est distant, le capteur non invasif 1 comprend des moyens de communication pour que le module de simulation 15 et le module d'adaptation 14 échangent des données.

**Module de simulation 15**

Le module de simulation 15 est un dispositif informatisé configuré pour générer un ensemble de configurations modèles correspondant (ou encore modélisant ou décrivant) chacune à un état particulier du milieu cible 2, un ensemble de cas d'irradiation du milieu cible 2 et les signaux photoacoustiques (ou le cas échéant photothermiques) théoriquement détectés en réponse à chaque cas d'irradiation pour chaque configuration modèle CMk du milieu cible 2 à partir de modèles analytiques du milieu cible 2 et de la cellule de détection photoacoustique (ou le cas échéant photothermique) 12, comme représenté sur la figure 4.

Ce module de simulation 15 est particulièrement pertinent dans le cas où le milieu cible 2 est évolutif au cours du temps et/ou stratifié. Dans ce cas, le milieu cible 2 adopte au cours du temps des configurations réelles différentes (une ou plusieurs concentrations varient au sein d'une ou plusieurs couches du milieu cible 2, une ou plusieurs dimensions telles que, par exemple, l'épaisseur d'une des couches du milieu cible 2)) qui peuvent chacune être modélisée par une configuration modèle particulière.

a) Modèle analytique multiphysique du milieu cible 2

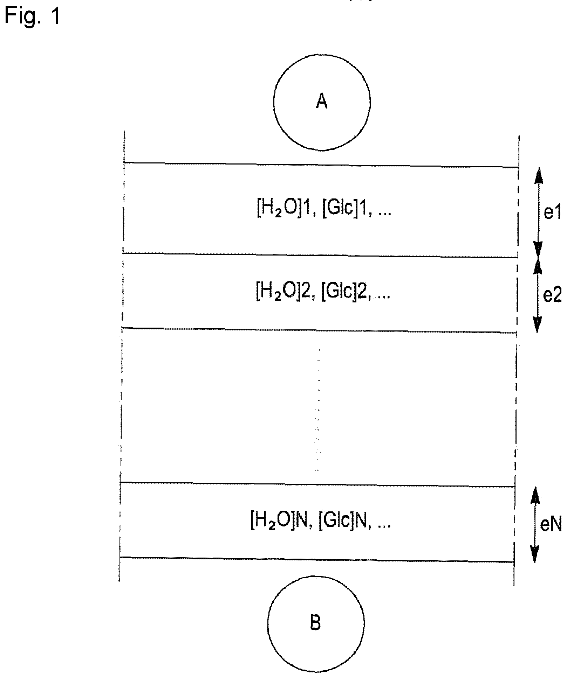

Le milieu cible 2 à analyser est modélisé comme représenté sur la figure 1. Le milieu cible 2 sépare un milieu extérieur A d'un milieu intérieur B et est composé d'une succession de N couches dont les interfaces sont par exemple supposées localement planes. Chaque couche $i$ $(i \in [\![1, N]\!])$ est décrite par le(s) paramètres d'intérêt et un certain nombre de paramètres explicites (appelés paramètres de niveau 1 car leur valeurs seront fournies en entrée du module de simulation 15 pour chaque simulation) appropriés pour le milieu cible 2 considéré. A titre d'exemple, si le milieu cible 2 est la peau et que l'on cherche à mesurer une concentration en glucose dans une couche j de ce milieu cible 2, le milieu cible 2 sera décrit par son nombre de couches N, la concentration [Glc]j d'intérêt, et chaque couche i peut être décrite pour la modélisation par son épaisseur ei, sa concentration en eau $[H_2O]i$. On peut éventuellement tenir compte des concentrations en glucose [Glc]i pour i≠j. Dans ce cas les valeurs de ei ( $i \in [\![1, N]\!]$ ), les concentrations $[H_2O]i$ ( $i \in [\![1, N]\!]$ ) et les concentrations [Glc]i pour ( $i \in [\![1, N]\!]$ , i≠j) sont les paramètres de niveau 1.

La liste des paramètres de niveau 1 peut être enrichie si l'on souhaite réaliser une modélisation plus précise. Notamment, les concentrations d'autres composants de la peau tels que les graisses, le lactate, l'oxygène, etc., peuvent être incluses dans la liste des paramètres de niveau 1 décrivant une couche de la peau.

Toujours pour l'exemple de la peau, on peut envisager de tenir compte de la couleur de la peau, de l'âge du patient, ou de tout autre paramètre anthropométrique, de manière à élargir ou restreindre l'espace des modèles possibles.

Des paramètres non explicites du modèle de milieu cible 2 (appelés paramètres de niveau 2 car non fournis en entrée du module de simulation 15) peuvent être calculés au moyen de modèles analytiques. Par exemple, la conductivité thermique, la capacité thermique, la densité ou encore le coefficient d'absorption à chaque longueur d'onde de chaque couche du milieu cible 2 peuvent être déduits des paramètres de niveau 1 et d'équations connues.

Le paramètre d'intérêt jouant un rôle particulier, il n'est pas inclus dans la liste des paramètres de niveau 1. Suivant l'étape du procédé, ce paramètre d'intérêt sera connu ou pas : sa valeur est connue pour procéder aux simulations au moyen du module de simulation 15, mais elle est bien sûr inconnue dans le cas d'une mesure réelle avec le capteur non invasif 1.

Le nombre de couches N du milieu cible 2 peut aussi être une variable du modèle. Toujours dans l'exemple de la peau, suivant les situations physiologiques, N peut ainsi être supérieur ou égal à un ou à deux. Ainsi, pour certaines situations physiologiques, la peau pourra être correctement décrite par deux couches, la première correspondant au stratum corneum, dont la concentration en glucose peut par exemple être faible, et la deuxième au reste de la peau, la concentration en glucose de la seconde couche étant assimilée à la concentration en glucose interstitielle à mesurer.

Pour d'autres situations, un modèle à trois couches ou plus sera plus adapté. Dans ce dernier cas, la concentration en eau d'une couche pourra par exemple augmenter avec la profondeur à laquelle se trouve cette couche.

N peut donc ne pas être une constante.

Dans le cas de la peau, le milieu extérieur A est en général l'atmosphère entourant le patient, qui remplit aussi la cellule de détection photoacoustique.

Le module de simulation 15 implémente un modèle de milieu stratifié 2 analytique multiphysique basé par exemple sur des équations physiques et/ou chimiques telles que, à titre d'exemple non limitatif, les équations de Beer-Lambert pour l'absorption optique et les équations thermodynamiques de la chaleur (loi de Fourier et lois de conservation).

Une configuration modèle CMk (k entier positif) de milieu cible 2 correspond à (ou encore modélise) un état particulier du milieu cible 2 donné. Cet état particulier est supposé correctement représenté par la donnée du nombre de couches N et des valeurs des paramètres de niveau 1 pour chaque couche.

Pour chaque configuration modèle CMk, le modèle analytique multiphysique permet, si le paramètre d'intérêt est de plus connu, de simuler l'onde thermique générée à l'interface couche 1/ milieu extérieur A (interface 1/A) en réponse une irradiation par une source de lumière 11a dont les paramètres d'irradiation sont connus, à savoir par exemple la fréquence de modulation fmod, la longueur d'onde λ et la densité surfacique de puissance.

En variante, le modèle analytique multiphysique permet de simuler l'onde de pression générée dans le milieu extérieur A.

Dans les deux cas, le signal obtenu en sortie d'un processeur implémentant le modèle analytique multiphysique est appelé « onde réponse simulée »

L'onde réponse simulée peut être fournie en entrée d'un processeur implémentant le modèle de cellule de détection.

### b) Modèle de cellule de détection

Le capteur non invasif 1 basé sur la détection photoacoustique ou photothermique comprend une cellule de détection photoacoustique (respectivement photothermique) 12 configurée pour détecter et analyser l'onde de pression (respectivement l'onde thermique) générée dans le milieu extérieur A lorsque l'onde thermique générée dans le milieu cible 2 en réponse à l'irradiation parvient à l'interface 1/A.

L'ensemble de la cellule de détection 12 peut être modélisé analytiquement. A partir d'une onde réponse simulée par le modèle analytique multiphysique, qui serait théoriquement reçue en entrée de la cellule de détection 12, le modèle de cellule de détection 12 permet de prédire le signal de sortie de la cellule de détection 12.

Différents modèles peuvent être envisagés.

Ainsi, dans le cas de la détection photoacoustique indirecte, les paramètres du modèle de la cellule de détection 12, appelés paramètres de cellule dans la suite, peuvent comprendre ses dimensions (par exemple la taille de l'évent, la hauteur de la cellule,...), des paramètres d'état thermodynamiques (température, pression atmosphérique, humidité relative ou absolue, ...). La cellule de détection photoacoustique peut notamment être modélisée au moyen d'un circuit RLC équivalent. A titre d'exemple, le modèle décrit dans Dehe, Alfons et al. "The Infineon Silicon MEMS Microphone." (2013) peut convenir.

Il est possible d'inclure dans ce modèle de cellule de détection photoacoustique un modèle de l'étape de traitement du signal effectuée par le module de traitement du signal 13 le cas échéant, de manière à générer, à partir de chaque onde réponse simulée générée par le processeur qui implémente le modèle multiphysique analytique, le signal théoriquement obtenu en sortie de cellule de détection photoacoustique (et le cas échéant après traitement du signal par le module de traitement du signal 13) qui lui correspond.

Le processeur du module de simulation 15 peut être configuré pour implémenter le modèle de cellule de détection photoacoustique.

Grâce au modèle analytique multiphysique de milieu cible 2 et au modèle de cellule de détection 12, on dispose donc d'un modèle analytique global qui permet à partir de la donnée de la configuration modèle CMk de milieu cible 2 et du cas d'irradiation Ij, à condition de fournir en outre le paramètre d'intérêt (qui peut effectivement être choisi puisqu'il s'agit d'une simulation), de prévoir le signal attendu en sortie de la cellule de détection 12 ou, le cas échéant, du module de traitement du signal 13. Ceci est représenté sur la figure 4.

En résumé, le module de simulation 15 reçoit donc en entrée les paramètres de la configuration modèle CMk de milieu cible 2, c'est-à-dire le nombre de couches N du milieu cible 2 et les paramètres de niveau 1 pour chaque couche, ainsi que le paramètre d'intérêt et les paramètres d'irradiation du cas d'irradiation Ij. En sortie, le module de simulation 15 fournit le signal théoriquement attendu en sortie de la cellule de détection 12 ou le cas échéant, le signal simulé traité théoriquement attendu en sortie du module de traitement du signal 13 pour la configuration modèle CMk de milieu cible 2 choisie, appelé signal de sortie simulé.

Le signal de sortie simulé peut être stocké en mémoire sous la forme d'un spectre de Fourier.

Les multiplets {configuration modèle CMk de milieu cible 2, cas d'irradiation Ij, paramètre d'intérêt, amplitudes et phases des composantes du signal de sortie simulé} peuvent être stockés dans une base de données de configurations modèles.

## Base de données de configurations modèles

On peut donc générer, éventuellement de manière automatisée et/ou aléatoire, un grand nombre de configurations modèles CMk, chaque configuration modèle CMk correspondant à un nombre de couches N et un jeu de paramètres de niveau 1, et optionnellement à une valeur ou une gamme de valeurs du paramètre d'intérêt, décrivant une situation particulière du milieu cible 2 d'intérêt.

Pour chaque configuration modèle CMk, on peut générer, éventuellement de manière automatisée et/ou aléatoire un grand nombre de cas d'irradiations Ij, chaque cas d'irradiation correspondant à un jeu de paramètres d'irradiation décrivant les paramètres de la (des) sources de lumière 11a utilisée(s) pour l'irradiation.

Un cas d'irradiation Ij peut donc comprendre une ou plusieurs fréquences de modulation de l'intensité d'un ou plusieurs lasers, la longueur d'onde de chacun de ces lasers et optionnellement la puissance irradiée par chaque laser.

Au moyen du module de simulation 15, on calcule l'amplitude et la phase de chaque composante du signal de sortie simulé obtenu en sortie du processeur du module de simulation 15 implémentant le modèle analytique global comprenant en cascade le modèle analytique multiphysique et le modèle de cellule de détection pour chaque configuration modèle CMk pour chaque cas d'irradiation Ij, une valeur du paramètre d'intérêt étant de plus fournie.

Les cas d'irradiation Ij peuvent être les mêmes pour plusieurs configurations modèles CMk différentes, et éventuellement plusieurs valeurs du paramètre d'intérêt, ou différents d'une configuration modèle CMk à une autre et/ou d'une valeur du paramètre d'intérêt à une autre. Une fois les simulations réalisées, on peut stocker dans une base de données de configurations modèles l'ensemble de ces configurations modèles CMk, cas d'irradiations, paramètres d'intérêt et signaux photoacoustiques (ou le cas échéant photothermiques) simulés associés sous forme de multiplets {configuration modèle CMk, cas d'irradiation Ij, paramètre d'intérêt, amplitude et phase des composantes du signal de sortie simulé}

La génération des configurations modèles CMk et/ou des cas d'irradiation Ij peut ne pas être complètement aléatoire.

La génération des configurations modèles CMk peut, entre autres, être fondée sur des considérations physiologiques pour restreindre l'espace des possibles à des configurations modèles physiologiquement réalistes. On peut par exemple limiter les épaisseurs possibles de la première couche de la peau à la gamme [8 μm, 40 μm] qui est effectivement observée expérimentalement, et limiter les concentrations en eau de cette couche à une gamme restreinte pour chaque épaisseur, la concentration en eau du stratum corneum étant corrélée avec son épaisseur.

La génération des cas d'irradiation peut notamment tenir compte des limitations des sources de lumière 11a disponibles pour un capteur non invasif 1 donné en longueur d'onde et/ou en puissance, ou encore des gammes de fréquences de modulation pertinentes pour le type de milieu cible 2 à analyser, ou des longueurs d'onde pertinentes pour le ou les paramètres d'intérêt.

En variante, la base de données de configurations modèles peut ne comprendre que des multiplets {configuration modèle CMk, cas d'irradiation Ij, paramètre d'intérêt, amplitudes et phases des composantes du signal mesuré réellement} obtenus par des expériences en situation réelles ou comprendre à la fois de tels multiplets obtenus en situation réelle et des multiplets obtenus par simulation.

## Table de correspondance

A partir de la base de données de configurations modèles, on peut entraîner dans le module de simulation 15 un modèle d'intelligence artificielle.

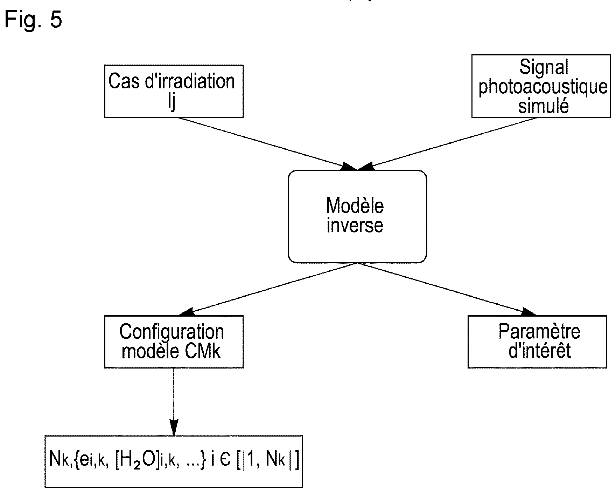

Le modèle d'intelligence artificielle, après apprentissage, est capable de résoudre le problème inverse, c'est-à-dire de retrouver le (ou les) paramètre(s) d'intérêt et la configuration modèle CMk de milieu cible 2, donc le nombre de couches N et les paramètres de niveau 1, connaissant le signal photoacoustique simulé et les paramètres d'irradiation du cas d'irradiation, comme représenté sur la figure 5.

Le modèle appris, qu'on appellera modèle inverse par la suite, peut être transmis au module d'adaptation 14 et stocké en mémoire de ce module.

Plusieurs modèles inverses différents peuvent être appris, avec des jeux d'apprentissage et/ou des règles d'apprentissage différents.

Dans la mesure où plusieurs cas d'irradiation Ij peuvent être associés à la même configuration modèle CMk, il est aussi possible par des techniques d'analyse statistique et/ou d'intelligence artificielle d'identifier le cas d'irradiation Ij qui permet de mesurer le paramètre d'intérêt avec la précision souhaitée et/ou la quantité de données de mesure permettant la consommation énergétique la plus faible.

Notamment, on peut procéder à une analyse de l'influence des variables (« feature importance ») dans le modèle entraîné. A l'issue de cette analyse, on peut associer à chaque configuration modèle CMk un cas d'irradiation I opt. k optimisé, comprenant un nombre restreint de longueurs d'onde d'irradiation et un nombre restreint de fréquences de modulation fmod pour chaque longueur d'onde d'irradiation.

Il est possible de travailler configuration modèle par configuration modèle ou bien de catégoriser les configurations modèles et d'associer à chaque catégorie de configurations modèles un cas d'irradiation I opt. cat.k optimal.

L'intérêt de la table de correspondance peut être compris à la lumière des figures 6a à 9b. Le contexte de ces figures est celui de la mesure de la glycémie interstitielle dans la peau. Des modèles inverse sont entraînés sur une base de données de configurations modèles comprenant, dans ce contexte, des multiplets (configuration modèle (CMk), cas d'irradiation (Ij), paramètre d'intérêt) et un signal acoustique ou thermique détecté par la cellule de détection associé à chaque multiplet.

Les données de la base de données ont été segmentées en deux groupes :

- le premier groupe « stratum épais » correspond à des configurations modèles de peau bicouches dans lesquelles l'épaisseur de la couche supérieure, modélisant le stratum corneum, est supérieure à 18 $\mu$m ;
- le second groupe « stratum fin » correspond à des configurations modèles de peau bicouches dans lesquelles l'épaisseur de la couche supérieure, modélisant le stratum corneum, est inférieure à 18 $\mu$m.

Un modèle inverse est entraîné pour chaque groupe à partir des signaux détectés pour six fréquences de modulation différentes. Puis une analyse de l'influence des variables est effectuée sur chacun des deux modèles inverses obtenus, comme représenté sur les figure 6a et 6b. Cette analyse de l'influence des variables permet de conclure que :

- pour les configurations modèles du groupe « stratum épais », les paramètres d'irradiation { longueur d'onde 1034 cm$^{-1}$ ; deux fréquences de modulation différentes : 50 Hz et 200Hz} sont ceux qui suffisent pour obtenir une mesure de glycémie à la précision souhaitée, l'analyse du module (équivalemment de l'amplitude) du signal détecté à chacune de ces fréquences étant suffisante ;

- pour les configurations modèles du groupe « stratum fin », les paramètres d'irradiation { longueur d'onde 1034 cm$^{-1}$ ; deux fréquences de modulation différentes : 50 Hz et 400Hz} et l'analyse du module (équivalemment de l'amplitude) du signal détecté à chacune de ces fréquences suffisent pour obtenir une mesure de glycémie à la précision souhaitée.

On comprend donc que le procédé permet de réduire la consommation énergétique pour chaque irradiation tout en contrôlant la précision de la mesure, grâce à une adaptation du cas d'irradiation à la configuration modèle détectée.

On pourra s'en convaincre en observant les résultats de mesure obtenu avec une irradiation complète, c'est à-dire dans ce cas suivant les six fréquences de modulation envisagées (figure 7a pour le groupe « stratum épais », figure 8a pour le groupe « stratum fin »), ou avec une irradiation limitée au cas d'irradiation optimal (figure 7b pour le groupe « stratum épais », figure 8b pour le groupe « stratum fin »).

Pour le groupe « stratum épais », la sélection de deux fréquences de modulation seulement parmi les six possibles (en l'occurrence 50 Hz et 200 Hz) et l'analyse uniquement de l'amplitude (ou équivalemment du module) de la composante du signal détectée à chacune de ces fréquences a conduit à :

- une variation relative de l'erreur quadratique moyenne de 7%,
- avec une réduction de la consommation d'un facteur au moins trois,

l'erreur quadratique moyenne passant de 10,5 mg/dL à 11,2 mg/dL, donc restant bien sous le seuil de consigne de 20 mg/dL considéré comme le RMSE maximal acceptable dans ce cas lors de la sélection des variables d'intérêt pour le cas d'irradiation optimal.

Pour le groupe « stratum fin », la sélection de deux fréquences de modulation seulement parmi les six possibles (en l'occurrence 50 Hz et 400 Hz) et l'analyse uniquement de l'amplitude (ou équivalemment du module) de la composante du signal détectée à chacune de ces fréquences a conduit à :

- une variation relative de l'erreur quadratique moyenne de 20%,
- avec une réduction de la consommation d'un facteur au moins trois,

l'erreur quadratique moyenne passant de 4,9 mg/dL à 5,8 mg/dL, donc restant bien sous le seuil de consigne de 20 mg/dL considéré comme le RMSE maximal acceptable dans ce cas lors de la sélection des variables d'intérêt pour le cas d'irradiation optimal. Même si la variation relative de l'erreur quadratique moyenne n'est pas négligeable dans ce cas, la valeur absolue de cette erreur quadratique moyenne reste donc contrôlée lors de la sélection du cas d'irradiation optimal.

En revanche, en l'absence d'association des configurations modèles à un cas d'irradiation particulier et à un modèle inverse particulier, c'est-à-dire dans le cas où un unique modèle inverse est entraîné pour l'ensemble des configurations modèles des deux groupes « stratum fin » et « stratum épais » et où l'irradiation est faite suivant un cas d'irradiation adapté à l'ensemble de ces configurations modèles des deux groupes « stratum fin » et « stratum épais », on voit que l'effet technique n'est pas obtenu.

Le cas d'irradiation optimal sélectionné pour l'ensemble des deux groupes peut être observé sur la figure 6c, pour lesquels les paramètres d'irradiation à sélectionner sont {longueur d'onde 1034 cm$^{-1}$ ; deux fréquences de modulation différentes : 200 Hz et 400Hz} et l'analyse du module (équivalemment de l'amplitude) du signal détecté à chacune de ces fréquences devant être effectuée.

Comme on peut l'observer sur les figures 9a et 9b, l'écart quadratique moyen est multiplié par un facteur trois lorsqu'on passe d'une irradiation suivant six fréquences de modulation différentes (RMSE = 13,0 mg/dL) à une irradiation suivant les deux fréquences de modulation identifiées comme les plus influentes (en l'occurrence 200 Hz et 400Hz) (RMSE = 26,9 mg/dL), avec analyse uniquement de l'amplitude du signal détecté à chacune de ces fréquences. La valeur seuil pour le RMSE, fixée pour cet exemple à 20 mg/dL, est d'ailleurs dépassée, indiquant que d'autres variables d'influence doivent être prises en compte pour respecter cette valeur seuil.

La table de correspondance peut éventuellement inclure un cas d'irradiation optimal pour l'ensemble des groupes, notamment en vue de l'irradiation initiale. Dans l'exemple qui précède, on comprend qu'il aurait été nécessaire d'inclure au moins une fréquence de modulation supplémentaire, à savoir 50 Hz, pour satisfaire le critère sur la valeur du RMSE pour l'irradiation initiale. La consommation aurait alors peut-être pu être réduite, mais seulement d'un facteur deux.

On voit donc sur les figures 7a à 9b que l'effet technique de réduction de la consommation à précision de mesure contrôlée est obtenu notamment au moyen de la table de correspondance, qui permet de segmenter l'ensemble des états possibles du milieu cible 2 en plusieurs catégories et d'associer un cas d'irradiation optimal pour chacune de ces catégories.

Dans ce cas, le nombre de paramètres d'irradiation (à savoir les deux grandeurs portant le plus d'information) a été sélectionné pour assurer que le RMSE reste inférieur à 20 mg/dL même avec une irradiation ne comprenant pas tous les possibles, et que la précision est meilleure que cette valeur seuil à basse glycémie, selon l'usage dans le domaine des capteurs de glycémie. Mais il serait possible de sélectionner 3, 4 ou plus de paramètres d'irradiation parmi tous les possibles pour assurer une valeur de RMSE inférieure à une autre valeur seuil, par exemple 15 mg/dL ou 10 mg/dL ou encore 5 mg/dL.

Le nombre de paramètres d'irradiation sélectionnés résulte d'un compromis entre la puissance disponible pour chaque irradiation et le RMSE acceptable.

Les couples {CMk ; I opt. k} ou {catégorie k de configurations modèles, Iopt.cat.k} sont stockés dans une table de correspondance en mémoire du capteur non invasif 1, par exemple dans une mémoire du module de simulation 15 et/ou du module d'adaptation 14.

Les modèles inverses correspondant à chaque configuration modèle CMk ou à chaque catégorie de configuration modèles sont aussi stockés en mémoire du capteur non invasif 1, par exemple dans une mémoire du module de simulation 15 et/ou du module d'adaptation 14.

A ce stade, les éléments nécessaires pour la mise en oeuvre du procédé de mesure suivant l'invention sont prêts.

On comprend d'ores et déjà que grâce à la table de correspondance, il n'est pas nécessaire lors d'une mesure d'établir un spectre optique complet de la cible pour l'état de la cible en cours de mesure : le choix automatique du cas d'irradiation optimal correspondant à la configuration modèle en cours, c'est-à-dire aux valeurs des paramètres de niveau 1 décrivant le mieux le milieu cible 2 pour la mesure en cours, pourra être effectué à partir de la table de correspondance.

Le procédé de mesure selon l'invention permet donc de réduire la consommation énergétique du capteur 1 par rapport aux procédés de l'art antérieur avec une précision identique voire améliorée.

**Procédé de mesure du paramètre d'intérêt**

Le procédé de mesure du paramètre d'intérêt au moyen du capteur non invasif 1 basé sur la détection photoacoustique indirecte est représenté sur la figure 2. Il comprend les étapes suivantes :

a) *Initialisation :*

On choisit une configuration modèle initiale de milieu cible 2 en vue de la première irradiation sur la base de critères prédéterminés. Par exemple, pour la figure 2, la configuration modèle CMirrad initiale est la configuration modèle CMk.

Différentes options peuvent être retenues pour cette initialisation.

Notamment, dans le cas de la mesure de la glycémie interstitiel à partir de la base de données de configuration modèles et/ou d'une base de données de mesures expérimentales, on peut déterminer un jeu de paramètres de niveau 1 et de glycémie moyen pour une population de patients ou pour un patient donné. Ce jeu de paramètres moyen correspond à une configuration modèle initiale CMk qui a la plus grande probabilité d'être la plus adaptée pour la mesure à venir en l'absence de toute autre information et notamment en l'absence d'un historique de mesure.

b) A partir de la table de correspondance, le processeur du module d'adaptation 14 détermine le cas d'irradiation Iopt.k associé à la configuration modèle initiale, par exemple CMk.

c) *Première irradiation :* On procède à une première irradiation du milieu cible 2 avec la ou les sources de lumière 11a suivant les paramètres d'irradiation du cas d'irradiation optimal Iopt.k pour la configuration modèle CMk.

d) *Détection PA :*
On détecte au moyen de la cellule de détection photoacoustique 12 le signal photoacoustique réel généré en réponse à l'irradiation. Le cas échéant, le module de traitement du signal 13 reçoit et traite ce signal photoacoustique réel et le transmet après traitement au module d'adaptation 14.

e) *Résolution du problème inverse :*
Le processeur du module d'adaptation 14 implémentant le(s) modèle(s) inverse appris (et notamment au moins celui correspondant à la configuration modèle CMk) reçoit en entrée le signal photoacoustique réel et le cas d'irradiation Iopt.k et détermine la configuration modèle de milieu cible 2 en cours, noté CMmes ainsi que le paramètre d'intérêt (comme représenté sur la figure 4).

f) *Validation de la configuration modèle :*

Le module d'adaptation 14 compare la configuration modèle de milieu cible 2 en cours CMmes à la configuration modèle CMirrad utilisée pour l'irradiation (configuration modèle CMk issue de l'étape initialisation pour la première mesure, configuration modèle CMℓ éventuellement différent pour une mesure ultérieure).
Cas 1) Si la configuration modèle de milieu cible 2 mesurée CMmes est identique à la configuration modèle CMirrad qui a été utilisée pour l'irradiation, les paramètres d'irradiation choisis étaient optimaux pour la situation physiologique en cours (qui on le rappelle n'est pas connue a priori et évolue au cours du temps), de même que le modèle inverse utilisé pour déterminer le paramètre d'intérêt. En conséquence, le paramètre d'intérêt déterminé à l'étape f par le module d'adaptation 14 est le résultat de la mesure.
Cas 2) Si la configuration modèle de milieu cible 2 mesurée CMmes est différente de la configuration modèle CMirrad, le processeur du module d'adaptation détermine au moyen de la table de correspondance une nouvelle configuration modèle CMℓ (ℓ≠k) mieux adaptée à la configuration modèle CMmes estimée par le module d'adaptation 14. Notamment, il est possible de choisir CMℓ = CMmes. Mais d'autres choix sont possibles, notamment si l'on veut tenir compte de l'historique des mesures sur une durée plus longue, en tenant compte d'au moins deux configurations modèles précédemment choisies.
Le processeur du module d'adaptation 14 recherche alors dans la table de correspondance le cas d'irradiation optimal Iopt. ℓ pour la configuration modèle de milieu cible 2 CMℓ et transmet les paramètres correspondants au dispositif d'irradiation 11.
Le milieu cible 2 subit alors une nouvelle irradiation (irradiation ultérieure) suivant les paramètres d'irradiation du cas d'irradiation Iopt. ℓ. Puis les étapes d) de détection et, le cas échéant de traitement du signal photoacoustique et e) de résolution du problème inverse sont réitérées.

Le procédé peut comprendre au plus une étape f) de validation de la configuration modèle.
En variante, le procédé peut comprendre aussi une réitération de l'étape f) de validation de la configuration modèle.
La configuration modèle de milieu cible 2 en cours CMmes peut être différente de la configuration modèle CMirrad. En effet, CMirrad a été choisie

- soit suivant les critères de l'initialisation (CMirrad est alors la configuration modèle « moyenne », notamment en l'absence d'historique de mesure sur le patient. C'est le cas pour la première irradiation). ;
- soit sur la base de la mesure précédente (CMirrad est alors configuration modèle la plus adaptée pour le patient connaissant le résultat de la mesure suite à l'irradiation précédente).

La validation de CMirrad se fait donc grâce à une information supplémentaire acquise grâce à l'irradiation en cours, à savoir le signal détecté par la cellule de détection photoacoustique 12. Si par hasard, CMmes = CMirrad, il n'est pas nécessaire de procéder à une nouvelle irradiation et la valeur du paramètre d'intérêt est bien la plus précise qui pouvait être obtenu, mais l'étape de validation de la configuration modèle a apporté une information supplémentaire à savoir la confirmation que la précision de la mesure est bien maximale pour ce cas.
Dans le cas, CMmes est différent de CMirrad, l'adaptation des paramètres d'irradiation et de la configuration modèle permet d'accroître la précision de la mesure au prix d'au moins une irradiation supplémentaire mais avec une consommation énergétique toujours maîtrisée, et grâce à la seconde étape de validation, de confirmer que la précision de la mesure est bien maximale. Dans le cas où l'on autorise la réitération de l'étape de validation (comme représenté sur la figure 2), on acquiert en sortie du procédé l'information supplémentaire que la précision de la mesure est bien maximale.

En général, dans ce cas, le résultat de mesure du paramètre d'intérêt est obtenu après la première irradiation ou après deux irradiations. Cependant, pour s'assurer que le procédé converge et/ou limiter la consommation énergétique, on peut prévoir une limitation du nombre d'étapes de validation de la configuration modèle.

Dans tous les cas, on constate que le choix d'un cas d'irradiation sur la base de la table de correspondance permet de limiter le nombre de fréquences de modulation et de longueurs d'onde utilisées pour l'irradiation en ne conservant que les valeurs porteuses d'informations non redondantes sur la configuration modèle de milieu cible 2 physiologique en cours et suffisantes pour obtenir la précision de mesure souhaitée et/ou optimales pour limiter la consommation en énergie du capteur à une valeur prédéterminée.

Par exemple, si la situation physiologique correspond à 3 couches chacune caractérisée par 2 concentrations (par exemple eau et glycémie), la résolution du problème inverse est un problème à $2*3 = 6$ inconnues. La connaissance de l'amplitude et la phase de chacune des trois composantes du signal photoacoustique correspondant à des fréquences de modulation et des longueurs d'onde bien choisies devrait permettre la résolution du problème inverse avec la précision souhaitée. La difficulté réside dans le choix optimal de ces fréquences de modulation et longueurs d'onde optimales, qui est résolu au moyen de la table de correspondance.

La consommation du capteur 1 est donc limitée ou contrôlée.

Même dans le cas où deux ou trois irradiations sont nécessaires, chaque étape d'irradiation nécessite une puissance limitée, inférieure à celle nécessaire pour obtenir un spectre d'absorption complet pour chaque fréquence de modulation possible.

L'étape de validation de la configuration modèle permet quant à elle :

- un recalibrage automatique du capteur 1 qui assure que le modèle inverse permettant de déduire une valeur du paramètre d'intérêt d'un signal photoacoustique détecté est le modèle inverse le plus pertinent pour la situation physiologique en cours, sans connaissance a priori de cette situation physiologique
- l'ajustement du cas d'irradiation, qui assure que les paramètres utilisés pour l'irradiation ultérieurs sont optimaux par rapport à la connaissance que l'on a du milieu cible et notamment celle apportée par le dernier signal photoacoustique détecté.

Plusieurs modes de réalisations peuvent être envisagés pour optimiser encore davantage le procédé de mesure suivant l'invention.

Notamment, le modèle d'intelligence artificielle du dispositif de simulation 15 peut être pré-entraîné sur la base de données de configurations modèles simulées puis ré-entraîné sur une base de données de configurations modèles expérimentales, correspondant à des situations réelles pour un patient donné ou un groupe de patients donnés, de manière à s'assurer que le modèle inverse prédit correctement les situations réelles sans pour autant que cela ne nécessite de disposer d'un jeu de données expérimentales exhaustif. L'apprentissage des modèles inverses peut comprendre dans ce cas une étape d'apprentissage par transfert (« transfert learning »).

## LISTE DES SIGNES DE RÉFÉRENCE

1 : capteur non invasif basé sur la détection photoacoustique indirecte
11 : dispositif d'irradiation
11a : source de lumière
11b : dispositif de modulation de l'intensité de la source de lumière 11a
11c : dispositif de contrôle de la fréquence de modulation fmod de l'intensité de la source de lumière 11a
12 : cellule de détection
13 : module de traitement du signal
14 : module d'adaptation
15 : module de simulation

## Revendications

1. Procédé de mesure d'un paramètre d'intérêt dans un milieu cible (2) au moyen d'un capteur non invasif (1) basé sur la détection photoacoustique ou la détection photothermique, comprenant :

a) on fournit un capteur comprenant :

- une source de lumière (11a),
- un dispositif de contrôle des paramètres d'irradiation de la source de lumière (1 1a),
- une cellule de détection configurée pour détecter un signal acoustique ou thermique (12),
- une mémoire dans laquelle est stockée une table de correspondance comprenant des configurations modèles (CMk) chacune représentative d'un état donné du milieu cible (2) et des cas d'irradiation optimaux (Iopt.k) comprenant chacun un jeu de paramètres d'irradiation, chaque configuration modèle (CMk) étant associée à un cas d'irradiation optimal,

- et un module d'adaptation (14) échangeant des informations avec la cellule de détection (12) et le dispositif de contrôle des paramètres d'irradiation de la source de lumière, ledit module d'adaptation (14) comprenant un processeur adapté pour implémenter un algorithme de modélisation inverse recevant en entrée un cas d'irradiation (Ij) comprenant un jeu de paramètres d'irradiation et un signal acoustique ou thermique et fournissant en sortie une configuration modèle (CMk) et une valeur du paramètre d'intérêt ;

b) le module d'adaptation (14) choisit une configuration modèle d'irradiation (CMirrad) initiale ;

c) le module d'adaptation (14) détermine dans la table de correspondance le cas d'irradiation optimal pour la configuration modèle d'irradiation choisie (CMirrad), c'est-à-dire le cas d'irradiation qui permet de mesurer le paramètre d'intérêt avec une précision prédéterminée et/ou la quantité de données de mesure permettant la consommation énergétique la plus faible ;

d) la source de lumière (11a) irradie le milieu cible (2) suivant le jeu de paramètres d'irradiation dudit cas d'irradiation optimal ;

e) la cellule de détection (12) détecte un signal acoustique ou thermique généré en réponse à l'irradiation ;

f) le processeur du module d'adaptation (14) implémente l'algorithme de modélisation inverse, reçoit en entrée le signal acoustique ou thermique détecté par la cellule de détection (12) et le cas d'irradiation optimal utilisé pour l'irradiation, et renvoie en sortie une configuration modèle en cours (CMmes) et une valeur du paramètre d'intérêt estimée (Pest) ;

g) le processeur du module d'adaptation (14) évalue la configuration modèle d'irradiation choisie (CMirrad) par comparaison avec la configuration modèle en cours (CMmes), et seulement si CMirrad est différent de CMmes : g1) le module d'adaptation (14) reçoit en entrée la configuration modèle en cours (CMmes) et renvoie en sortie une nouvelle configuration modèle en vue d'une irradiation (CMirrad), puis on réitère c), d), e) et f) ;

h) la valeur du paramètre d'intérêt mesurée (Pmes) par le capteur est la dernière valeur du paramètre d'intérêt estimée (Pest).

2. Procédé de mesure suivant la revendication 1, g1) comprenant en outre on réitère g) à l'issue de f).

3. Procédé de mesure suivant l'une des revendications 1 et 2 comprenant au préalable :

I- on génère une table de correspondance au moyen d'un processeur et d'une base de données de configurations modèles comprenant des multiplets (configuration modèle (CMk), cas d'irradiation (Ij), paramètre d'intérêt) et un signal acoustique ou thermique détecté par la cellule de détection (12) associé à chaque multiplet et on stocke cette table de correspondance dans la mémoire du capteur non invasif (1).

4. Procédé de mesure suivant la revendication 3 comprenant :
II- un processeur apprend au moins un algorithme de modélisation inverse à partir de la base de données de configurations modèles et on stocke l'au moins un algorithme de modélisation inverse dans la mémoire du capteur non invasif (1).

5. Procédé de mesure suivant l'une quelconque des revendications 3 à 4 dans lequel au moins une partie des signaux acoustiques ou thermiques détectés par la cellule de détection (12) associés aux multiplets stockés dans la base de données de configurations modèles sont simulés, c'est-à-dire qu'ils sont générés au moyen d'un dispositif informatisé de simulation.

6. Capteur non invasif (1) basé sur la détection photoacoustique ou photothermique configuré pour mesurer un paramètre d'intérêt dans un milieu cible (2) comprenant :

- une source de lumière (11a),
- un dispositif de contrôle des paramètres d'irradiation de la source de lumière (1 1a),
- une cellule de détection (12) configurée pour détecter un signal acoustique ou thermique,
- une mémoire dans laquelle est stockée une table de correspondance comprenant des configurations modèles (CMk) chacune représentative d'un état donné du milieu cible (2) et des cas d'irradiation optimaux (Iopt.k) comprenant chacun un jeu de paramètres d'irradiation, chaque configuration modèle (CMk) étant associée à un cas d'irradiation optimal (Iopt.k),

le capteur non invasif comprenant en outre un module d'adaptation (14) adapté pour échanger des informations avec la cellule de détection (12) et le dispositif de contrôle des paramètres d'irradiation de la source de lumière (11a) et qui comprend un processeur adapté pour implémenter un algorithme de modélisation inverse recevant en entrée un cas d'irradiation (Ij) comprenant un jeu de paramètres d'irradiation et un signal acoustique ou thermique et fournissant en sortie une configuration modèle (CMk) et une valeur du paramètre d'intérêt, le module d'adaptation (14) étant en outre configuré pour :

> i - choisir une configuration modèle d'irradiation (CMirrad) initiale,
> ii - déterminer dans la table de correspondance un cas d'irradiation optimal correspondant à une configuration modèle d'irradiation, c'est-à-dire le cas d'irradiation qui permet de mesurer le paramètre d'intérêt avec une précision prédéterminée et/ou la quantité de données de mesure permettant la consommation énergétique la plus faible
> iii - transmettre un cas d'irradiation optimal au dispositif de contrôle des paramètres d'irradiation de la source de lumière,
> iv - recevoir un signal détecté par la cellule de détection (12),
> v - déterminer une configuration modèle en cours (CMmes) et une valeur du paramètre d'intérêt estimée (Pest) sur la base d'un signal photoacoustique ou photothermique détecté reçu et d'un cas d'irradiation optimal,
> vi - évaluer une configuration modèle d'irradiation (CMirrad) par comparaison avec une configuration modèle en cours (CMmes),
> vii- seulement si la configuration modèle d'irradiation du milieu stratifié cible choisie (CMirrad) et la configuration modèle du milieu stratifié cible en cours (CMmes) comparées sont différentes, déterminer une nouvelle configuration modèle d'irradiation (CMirrad) lorsqu'il reçoit une configuration modèle en cours (CMmes), déterminer dans la table de correspondance un nouveau cas d'irradiation optimal correspondant à la nouvelle configuration modèle d'irradiation (Cmirrad) et transmettre le nouveau cas d'irradiation optimal au dispositif de contrôle des paramètres d'irradiation de sorte que la source de lumière irradie le milieu stratifié cible suivant le jeu de paramètres d'irradiation du nouveau cas d'irradiation optimal et que la cellule de détection détecte un nouveau signal thermique ou acoustique généré en réponse à cette irradiation et le transmette au processeur du module d'adaptation (14) configuré pour réitérer iv, v, vi et vii,

- déterminer la valeur du paramètre d'intérêt mesurée (Pmes) sur la base de la dernière valeur du paramètre d'intérêt estimée (Pest).

7. Programme d'ordinateur comprenant des instructions qui conduisent le capteur non invasif (1) suivant la revendication 6 à exécuter les étapes du procédé suivant l'une quelconque des revendications 1 à 5.

**Patentansprüche**

1. Verfahren zum Messen eines Parameters von Interesse in einem Zielmedium (2) mittels eines nichtinvasiven Sensors (1), der auf photoakustischer Detektion oder photothermischer Detektion basiert, das Folgendes beinhaltet:

> a) Bereitstellen eines Sensors, der Folgendes umfasst:
>
>> - eine Lichtquelle (11a),
>> - eine Vorrichtung zur Steuerung der Strahlungsparameter der Lichtquelle (11a),
>> - eine Detektionszelle, die zum Detektieren eines akustischen oder thermischen Signals (12) konfiguriert ist,
>> - einen Speicher, in dem eine Korrespondenztabelle gespeichert ist, die Modellkonfigurationen (CMk), die jeweils für einen gegebenen Zustand des Zielmediums (2) repräsentativ sind, und optimale Strahlungsfälle (Iopt.k) umfasst, die jeweils einen Satz von Strahlungsparametern umfassen, wobei jede Modellkonfiguration (CMk) mit einem optimalen Strahlungsfall assoziiert ist,
>> - und ein Anpassungsmodul (14), das Informationen mit der Detektionszelle (12) und der Vorrichtung zur Steuerung der Strahlungsparameter der Lichtquelle austauscht, wobei das genannte Anpassungsmodul (14) einen Prozessor umfasst, der zum Implementieren eines inversen Modellierungsalgorithmus ausgelegt ist, der als Eingabe einen Strahlungsfall (Ij), der einen Satz von Strahlungsparametern umfasst, und ein akustisches oder thermisches Signal empfängt und als Ausgabe eine Modellkonfiguration (CMk) und einen Wert des Parameters von Interesse liefert;

> b) Auswählen, durch das Anpassungsmodul (14), einer ursprünglichen Strahlungsmodellkonfiguration (CMirrad);
> c) Bestimmen, durch das Anpassungsmodul (14), in der Korrespondenztabelle, des optimalen Strahlungsfalls für die ausgewählte Strahlungsmodellkonfiguration (CMirrad), d.h. des Strahlungsfalls, der es ermöglicht, den Parameter von Interesse mit einer vorbestimmten Genauigkeit und/oder die Menge an Messdaten, die den geringsten Energieverbrauch zulässt, zu messen;

d) Bestrahlen, durch die Lichtquelle (11a), des Zielmediums (2) gemäß dem Satz von Strahlungsparametern des genannten optimalen Strahlungsfalls,

e) Detektieren, durch die Detektionszelle (12), eines als Reaktion auf die Strahlung erzeugten akustischen oder thermischen Signals;

f) durch den Prozessor des Anpassungsmoduls (14), Implementieren des inversen Modellierungsalgorithmus, Empfangen, als Eingabe, des von der Detektionszelle (12) detektierten akustischen oder thermischen Signals und des für die Strahlung verwendeten optimalen Strahlungsfalls und Zurückgeben, als Ausgabe, einer aktuellen Modellkonfiguration (CMmes) und eines geschätzten Wertes des Parameters von Interesse (Pest);

g) Bewerten, durch den Prozessor des Anpassungsmoduls (14), der ausgewählten Strahlungsmodellkonfiguration (CMirrad) durch Vergleichen mit der aktuellen Modellkonfiguration (CMmes), und nur dann, wenn CMirrad von CMmes verschieden ist: g1) durch das Anpassungsmodul (14), Empfangen, als Eingabe, der aktuellen Modellkonfiguration (CMmes) und Zurückgeben, als Ausgabe, einer neuen Modellkonfiguration im Hinblick auf eine Strahlung (CMirrad), woraufhin c), d), e) und f) wiederholt werden;

h) wobei der vom Sensor gemessene Wert des Parameters von Interesse (Pmes) der letzte geschätzte Wert des Parameters von Interesse (Pest) ist.

2. Messverfahren nach Anspruch 1, wobei g1) außerdem das Wiederholen von g) nach Abschluss von f) beinhaltet.

3. Messverfahren nach einem der Ansprüche 1 und 2, das zuvor Folgendes beinhaltet:

   I- Erstellen einer Korrespondenztabelle mittels eines Prozessors und einer Datenbank von Modellkonfigurationen, die Bytes (Modellkonfiguration (CMk), Strahlungsfall (Ij), Parameter von Interesse) und ein von der mit jedem Byte assoziierten Detektionszelle (12) detektiertes akustisches oder thermisches Signal umfasst, und Speichern dieser Korrespondenztabelle im Speicher des nichtinvasiven Sensors (1).

4. Messverfahren nach Anspruch 3, das Folgendes beinhaltet:
   II- Lernen, durch einen Prozessor, mindestens eines inversen Modellierungsalgorithmus auf der Basis der Modellkonfigurationsdatenbank und Speichern des mindestens einen inversen Modellierungsalgorithmus im Speicher des nichtinvasiven Sensors (1).

5. Messverfahren nach einem der Ansprüche 3 bis 4, wobei mindestens ein Teil der von der Detektionszelle (12) detektierten akustischen oder thermischen Signale, die mit den in der Modellkonfigurationsdatenbank gespeicherten Bytes assoziiert sind, simuliert wird, d.h. sie werden mithilfe einer computergestützten Simulationsvorrichtung erzeugt.

6. Nichtinvasiver Sensor (1), der auf photoakustischer oder photothermischer Detektion basiert und zum Messen eines Parameters von Interesse in einem Zielmedium (2) konfiguriert ist, der Folgendes umfasst:

   - eine Lichtquelle (11a),
   - eine Vorrichtung zur Steuerung der Strahlungsparameter der Lichtquelle (11a),
   - eine Detektionszelle (12), die zum Detektieren eines akustischen oder thermischen Signals konfiguriert ist,
   - einen Speicher, in dem eine Korrespondenztabelle gespeichert ist, die Modellkonfigurationen (CMk), die jeweils für einen gegebenen Zustand des Zielmediums (2) repräsentativ sind, und optimale Strahlungsfälle (Iopt.k) umfasst, die jeweils einen Satz von Strahlungsparametern umfassen, wobei jede Modellkonfiguration (CMk) mit einem optimalen Strahlungsfall (Iopt.k) assoziiert ist,

   wobei der nichtinvasive Sensor außerdem ein Anpassungsmodul (14) umfasst, das zum Austauschen von Informationen mit der Detektionszelle (12) und der Vorrichtung zur Steuerung der Strahlungsparameter der Lichtquelle (11a) ausgelegt ist, und das einen Prozessor umfasst, der zum Implementieren eines inversen Modellierungsalgorithmus ausgelegt ist, der als Eingabe einen Strahlungsfall (Ij), der einen Satz von Strahlungsparametern umfasst, und ein akustisches oder thermisches Signal empfängt und als Ausgabe eine Modellkonfiguration (CMk) und einen Wert des Parameters von Interesse liefert, wobei das Anpassungsmodul (14) außerdem konfiguriert ist zum:

   i - Auswählen einer ursprünglichen Strahlungsmodellkonfiguration (CMirrad),

ii - Bestimmen, in der Korrespondenztabelle, eines optimalen Strahlungsfalls, der einer Strahlungsmodellkonfiguration entspricht, d.h. des Strahlungsfalls, der es ermöglicht, den Parameter von Interesse mit einer vorbestimmten Genauigkeit und/oder die Menge an Messdaten, die den geringsten Energieverbrauch zulässt, zu messen,

iii - Senden eines optimalen Strahlungsfalls zu der Vorrichtung zur Steuerung der Strahlungsparameter der Lichtquelle,

iv - Empfangen eines von der Detektionszelle (12) detektierten Signals,

v - Bestimmen einer aktuellen Modellkonfiguration (CMmes) und eines geschätzten Wertes des Parameters von Interesse (Pest) auf der Basis eines empfangenen detektierten photoakustischen oder photothermischen Signals und eines optimalen Strahlungsfalls,

vi - Bewerten einer Strahlungsmodellkonfiguration (CMirrad) durch Vergleichen mit einer aktuellen Modellkonfiguration (CMmes),

vii - nur dann, wenn die ausgewählte Strahlungsmodellkonfiguration des geschichteten Zielmediums (CMirrad) und die aktuelle Modellkonfiguration des geschichteten Zielmediums (CMmes), die verglichen wurden, unterschiedlich sind, Bestimmen einer neuen Strahlungsmodellkonfiguration (CMirrad), wenn es eine aktuelle Modellkonfiguration (CMmes) empfängt, Bestimmen, in der Korrespondenztabelle, eines neuen optimalen Strahlungsfalls, der der neuen Strahlungsmodellkonfiguration (Cmirrad) entspricht, und Senden des neuen optimalen Strahlungsfalls zu der Vorrichtung zur Steuerung der Strahlungsparameter, so dass die Lichtquelle das geschichtete Zielmedium gemäß dem Satz von Strahlungsparametern des neuen optimalen Strahlungsfalls bestrahlt und die Detektionszelle ein neues thermisches oder akustisches Signal detektiert, das als Reaktion auf diese Bestrahlung erzeugt wird, und es zum Prozessor des Anpassungsmoduls (14) sendet, der zum Wiederholen von iv, v, vi und vii konfiguriert ist,

- Bestimmen des gemessenen Wertes des Parameters von Interesse (Pmes) auf der Basis des letzten geschätzten Wertes des Parameters von Interesse (Pest).

7. Computerprogramm mit Befehlen, die den nichtinvasiven Sensor (1) nach Anspruch 6 zum Ausführen der Schritte des Verfahrens nach einem der Ansprüche 1 bis 5 veranlassen.

**Claims**

1. A method of measuring a parameter of interest in a target environment (2) by means of a non-invasive sensor (1) based on photoacoustic detection or photothermal detection, comprising:

    a) a sensor is provided comprising:

        - a light source (11a),
        - a device for controlling the irradiation parameters of the light source (11a),
        - a detection cell configured to detect an acoustic or thermal signal (12),
        - a memory in which is stored a correspondence table comprising model configurations (CMk) each representative of a given state of the target environment (2) and optimal irradiation cases (Iopt.k) each comprising a set of irradiation parameters, each model configuration (CMk) being associated with an optimal irradiation case,
        - and an adaptation module (14) exchanging information with the detection cell (12) and the light source irradiation parameter control device, said adaptation module (14) comprising a processor adapted to implement an inverse modeling algorithm receiving as input an irradiation case (Ij) comprising a set of irradiation parameters and an acoustic or thermal signal and providing as output a model configuration (CMk) and a value of the parameter of interest;

    b) the adaptation module (14) selects an initial irradiation model configuration (CMirrad);
    c) the adaptation module (14) determines from the correspondence table the optimum irradiation case for the chosen irradiation model configuration (CMirrad), i.e. the irradiation case that allows the parameter of interest to be measured with a pre-determined accuracy and/or the quantity of measurement data allowing the lowest energy consumption;
    d) the light source (11a) irradiates the target environment (2) according to the set of irradiation parameters of said optimal irradiation case;
    e) the detection cell (12) detects an acoustic or thermal signal generated in response to irradiation;

f) the adaptation module processor (14) implements the inverse modeling algorithm, receives as input the acoustic or thermal signal detected by the detection cell (12) and the optimal irradiation case used for irradiation, and returns as output a current model configuration (CMmes) and an estimated value of the parameter of interest (Pest);

g) the processor of the adaptation module (14) evaluates the chosen irradiation model configuration (CMirrad) by comparison with the current model configuration (CMmes), and only if CMirrad is different from CMmes: g1) the adaptation module (14) receives as input the current model configuration (CMmes) and returns as output a new model configuration for irradiation (CMirrad), then c), d), e) and f) are repeated;

h) the value of the parameter of interest measured (Pmes) by the sensor is the last value of the parameter of interest estimated (Pest);

2. Measurement method according to claim 1, g1) further comprising repeating g) after f);

3. Measuring method according to one of claims 1 and 2 comprising beforehand:

I- a correspondence table is generated by means of a processor and a database of model configurations comprising multiplets (model configuration (CMk), irradiation case (Ij), parameter of interest) and an acoustic or thermal signal detected by the detection cell (12) associated with each multiplet, and this correspondence table is stored in the memory of the non-invasive sensor (1);

4. Measurement method according to claim 3 comprising :

II- a processor learns at least one inverse modeling algorithm from the database of model configurations and the at least one inverse modeling algorithm is stored in the memory of the non-invasive sensor (1);

5. A measurement method according to any one of claims 3 to 4 in which at least some of the acoustic or thermal signals detected by the sensor cell (12) associated with the multiplets stored in the model configuration database are simulated, i.e. generated by means of a computerized simulation device;

6. A non-invasive sensor (1) based on photoacoustic or photothermal detection configured to measure a parameter of interest in a target environment (2) comprising:

- a light source (11a),
- a device for controlling the irradiation parameters of the light source (11a),
- a detection cell (12) configured to detect an acoustic or thermal signal,
- a memory in which is stored a correspondence table comprising model configurations (CMk) each representative of a given state of the target environment (2) and optimal irradiation cases (Iopt.k) each comprising a set of irradiation parameters, each model configuration (CMk) being associated with an optimal irradiation case (Iopt.k),

the non-invasive sensor further comprising an adaptation module (14) adapted to exchange information with the detection cell (12) and the light source for controlling the irradiation parameters of the light source (11a) and which comprises a processor adapted to implement an inverse modeling algorithm receiving as input an irradiation case (Ij) comprising a set of irradiation parameters and an acoustic or thermal signal and providing as output a model configuration (CMk) and a value of the parameter of interest,

the adaptation module (14) being further configured to :

i- select an initial model irradiation configuration (CMirrad),

ii- determine an optimal irradiation case corresponding to an irradiation model configuration from the correspondence table, i.e. the irradiation case that allows the parameter of interest to be measured with a pre-determined accuracy and/or the quantity of measurement data allowing the lowest energy consumption;

iii- transmit an optimal irradiation case to the light source irradiation parameter control device,

iv- receive a signal detected by the detection cell(12),

v- determine a current model configuration (CMmes) and an estimated value of the parameter of interest (Pest) on the basis of a detected photoacoustic or photothermal signal received and an optimal irradiation case,

vi- evaluate a model irradiation configuration (CMirrad) by comparison with a current model configuration (CMmes),

vii- only if the model irradiation configuration of the selected target stratified environment (CMirrad) and the model configuration of the current target stratified environment (CMmes) compared are different, determine a new model irradiation configuration (CMirrad) when it receives a current model configuration (CMmes), determine a new optimum irradiation case from the look-up table, corresponding to the new irradiation model configuration (Cmirrad), and transmit the new optimum irradiation case to the irradiation parameter control device, so that the light source irradiates the target stratified environment according to the irradiation parameter set of the new optimum irradiation case, and the detection cell detects a new thermal or acoustic signal generated in response to this irradiation, and transmits it to the processor of the adaptation module (14) configured to reiterate iv, v, vi and vii;

- determine the value of the parameter of interest measured (Pmes) on the basis of the last estimated value of the parameter of interest (Pest);

7. A computer program comprising instructions which lead the non-invasive sensor (1) according to claim 6 to perform the steps of the method according to any one of claims 1 to 5.

Fig. 1

Fig. 2

**Initialisation** : choix de la configuration modèle initiale
CMirrad = CMk
$N_k, \{e_{i,k}, [H_2O]_{i,k}, ...\}$ i ∈ [|1, $N_k$|]
(sauf [Glc])

**Irradiation initiale** suivant les paramètres d'irradiation du cas
d'irradiation optimal Iopt.k pour la configuration modèle
initiale CMk
$\{f_{mod,p,k}; \lambda_{p,k}\}$ p ∈ [|1, $p_k$|]

**Détection PA**
Aplitude et phase pour
tous les couples $\{f_{mod,p}; \lambda_p\}$ de
la configuration modèle choisie

**Irradiation ultérieure** suivant les
paramètres optimaux pour
CMirrad = CMℓ :
$\{f_{mod,p,\ell} ; \lambda_{p,\ell}\}$ p ∈ [|1, $p_\ell$|]

**Résolution du problème inverse**
Détermination de la configuration
modèle CMmes correspondant
au signal photoacoustique ou
photothermique détecté

**Choix** d'une nouvelle
configuration modèle
CMirrad = CMℓ

**Validation** de la
configuration modèle :
CMmes = CMirrad?

non

oui

[Glc] estimée

## Fig. 3

## Fig. 4

Fig. 5

Cas d'irradiation Ij

Signal photoacoustique simulé

Modèle inverse

Configuration modèle CMk

Paramètre d'intérêt

$N_k, \{e_{i,k}, [H_2O]_{i,k}, ...\}\ i \in [|1, N_k|]$

Fig. 6a

EP 4 245 218 B1

Fig. 6b

## Fig. 6c

Fig. 7a

Erreur quadratique moyenne: 10.5 mg/dL

Fig. 7b

Erreur quadratique moyenne: 11.2 mg/dL

Fig. 8a

Erreur quadratique moyenne: 4.9 mg/dL

Fig. 8b

Erreur quadratique moyenne: 5.8 mg/dL

**Fig. 9a**

Erreur quadratique moyenne: 13.0 mg/dL

Valeur prédite [mg/dL] vs Valeur attendue [mg/dL]

**Fig. 9b**

Erreur quadratique moyenne: 26.9 mg/dL

Valeur prédite [mg/dL] vs Valeur attendue [mg/dL]

**EP 4 245 218 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2460470 A

**Littérature non-brevet citée dans la description**

- **ROSENCWAIG, A. ; GERSHO, A.** Theory of the photoacoustic effect with solids. *Journal of Applied Physics,* 1976, vol. 47, 64

- **HU, H. ; WANG, X ; XU, X.** Generalized theory of the photoacoustic effect in a multilayer material. *Journal of Applied Physics,* 1999, vol. 86, 3953-3958
- **DEHE, ALFONS et al.** *The Infineon Silicon MEMS Microphone,* 2013